(19) Europäisches Patentamt
European Patent Office
Office européen des brevets

(11) Numéro de publication: **0 240 398 B1**

(12) # FASCICULE DE BREVET EUROPEEN

(45) Date de publication de fascicule du brevet: **15.05.91**

(51) Int. Cl.[5]: **C07D 491/10**, C07D 211/90, A61K 31/44, //(C07D491/10, 317:00,221:00)

(21) Numéro de dépôt: **87400598.6**

(22) Date de dépôt: **18.03.87**

Le dossier contient des informations techniques présentées postérieurement au dépôt de la demande et ne figurant pas dans le présent fascicule.

(54) **Dérivés dissymétriques de l'acide 1,4-dihydropyridine-3,5-dicarboxylique, procédés de préparation et utilisation en thérapeutique.**

(30) Priorité: **02.04.86 FR 8604685**

(43) Date de publication de la demande: **07.10.87 Bulletin 87/41**

(45) Mention de la délivrance du brevet: **15.05.91 Bulletin 91/20**

(84) Etats contractants désignés: **AT BE CH DE ES FR GB GR IT LI LU NL SE**

(56) Documents cités:
**EP-A- 0 088 903**
**EP-A- 0 194 906**
**FR-M- 7 896**

**CHEMICAL ABSTRACTS, vol. 82, no. 21, 26 mai 1975, page 627, résumé no. 140108j, Columbus, Ohio, US; NAKANISHI M. et al.: "1,4-Dioxa-8-azaspiro[4.5]decane derivative"; & JP-A-74 32 542**

**CHEM. PHARM. BULL. 33(9), page 3787, 1985**

(73) Titulaire: **FOURNIER INNOVATION ET SYNERGIE**
**38, avenue Hoche**
**F-75008 Paris(FR)**

(72) Inventeur: **Robin, Jacques**
**19, rue de la Corvée**
**F-21000 Dijon(FR)**
Inventeur: **Pruneau, Didier**
**Rue Haute,Baulme la Roche**
**F-21410 Pont de Pany(FR)**
Inventeur: **Bellamy, François**
**Rue Basse Cédex 11**
**F-21910 Saulon la Rue(FR)**

(74) Mandataire: **Clisci, Serge et al**
**S.A. FEDIT-LORIOT CONSEILS EN PROPRIETE INDUSTRIELLE 38, avenue Hoche**
**F-75008 Paris(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services

ANNUAL REPORTS IN MEDICINAL CHEMISTRY, vol. 23, page 64, Academic Press, 1988, Baldwin et al.

CHEMICAL ABSTRACTS, vol. 97, 1982, résumé 120317h; TAKENAKA T. et al.: "Vasodilator and hypotensive effects of the optical isomers of nicardipine (YC-93), a new calcium (2+)-antagonist"

EUROPEAN JOURNAL OF PHARMACOLOGY, vol. 146, pages 171-174, 1988; BRISAC A.M. et al.: "Central and peripheral hypotensive effects of the optical isomers of nicardipine, a dihydropyridine calcium channel antagonist, in rats"

## Description

La présente invention a trait à de nouveaux dérivés de l'acide 1,4-dihydropyridine-3,5-dicarboxylique de formule I ci-après. Elle concerne également leurs procédés de préparation et leur application en thérapeutique.

On sait que FR-M-7 896 a trait à des homologues supérieurs de la nifédipine en tant qu'agents coronarodilatateurs utiles notamment dans le traitement de l'angine de poitrine. Dans la formule de ces homologues, les restes $CH_3$ des fonctions 3,5-dicarboxylate de méthyle de la nifédipine sont remplacés par un reste alkyle ou alkylène ayant au plus 6 atomes de carbone et interrompu notamment par un ou plusieurs atomes d'oxygène. La formule développée du document FR-M-7 896 n'englobe pas les composés de formule I selon la présente invention.

Le document EP-A-0 088 903 a trait à des dérivés de la nifédipine et de la nicardipine en tant qu'agents antihypertenseurs et vasodilatateurs utiles dans le traitement des troubles cardiaques et cérébraux de la circulation. Ces dérivés présentent en position 3 du cycle 1,4-dihydropyridine un reste ester, tel que notamment le reste carboxylate de méthyle, et en position 5 dudit cycle 1,4-dihydropyridine un reste

$$COO-CHZ-(CH_2)_n-A \qquad (Q)$$

où Z est un groupe aryle ou un reste hétéroaromatique de 5 à 6 sommets, n est un nombre entier ayant pour valeurs 1 à 5, et A est un reste N-hétérocyclique de 5 à 7 sommets pouvant contenir un second hétéroatome tel que N, S et O et susceptible d'être substitué, notamment par un groupe éthylènedioxy. Il est enseigné (cf. page 3, lignes 13-17, de EP-A-0 088 903) que la présence d'un groupe Z aromatique ou hétéroaromatique en position $\alpha$ sur le reste Q améliore et prolonge l'efficacité. Le produit de l'exemple 22 de EP-A-0 088 903 comporte effectivement un groupe éthylènedioxy sur le cycle A du reste Q; ce produit qui a pour formule développée

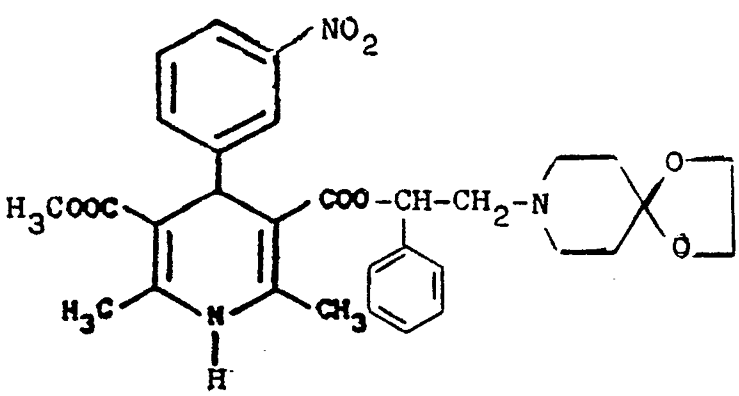

ne décrit ni ne suggère les composés de formule I selon l'invention eu égard (i) à la position du groupe éthylènedioxy et surtout (ii) à la présence d'un groupe aromatique en position $\alpha$ sur sa chaîne 5-ester.

On sait par ailleurs que l'article de K. MEGURO et al., Chem. Pharm. Bull., 33 (No. 9), pages 3787-3797 (1985) signale que pour la durée d'action de la nicardipine et de la nifédipine est trop courte et préconise de modifier la chaîne latérale de la nicardipine en vue d'augmenter ladite durée d'action (voir page 3787 lignes 4-6, à tableau I page 3791 dudit article).

La demande de brevet français FR-A-2 577 552 (qui correspond à EP-A-0 194 906) de la demanderesse décrit des esters hétérocycliques dissymétriques d'acides 1,4-dihydropyridine-3,5-dicarboxylique qui répondent à la formule

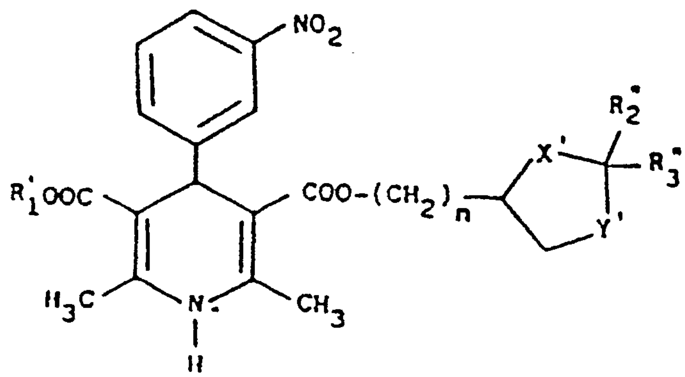

(Io)

où notamment R'₁ est alkyle en $C_1$-$C_4$, n est un entier valant 1 ou 2, X' et Y' représentent chacun O ou S, un au moins des X' et Y' étant différent de S, et R"₂ et R"₃ représentent chacun l'atome d'hydrogène, le groupe méthyle, les groupes phényle ou halogénophényle, ou forment ensemble un groupe spirocycloaliphatique.

Les produits selon FR-A-2 577 552 et EP-A-0 194 906 sont indiqués en tant qu'agents vasodilatateurs et se distinguent des produits connus de l'art antérieur, notamment par leurs effets antihypertenseurs et leurs effets sur les débits cardiaque, fémoral et coronaire.

La demanderesse a cherché à optimiser les propriétés pharmacologiques de ces derniers produits et vient à présent de trouver de nouveaux dérivés de l'acide 1,4-dihydropyridine-3,5-dicarboxylique qui se distinguent des produits de l'art antérieur, notamment par leur structure et de la demande FR-A-2 577 552 précitée par leur activité par voie orale.

Les nouveaux dérivés selon l'invention sont caractérisés en ce qu'ils sont choisis parmi l'ensemble constitué par

(i) les esters répondant à la formule générale :

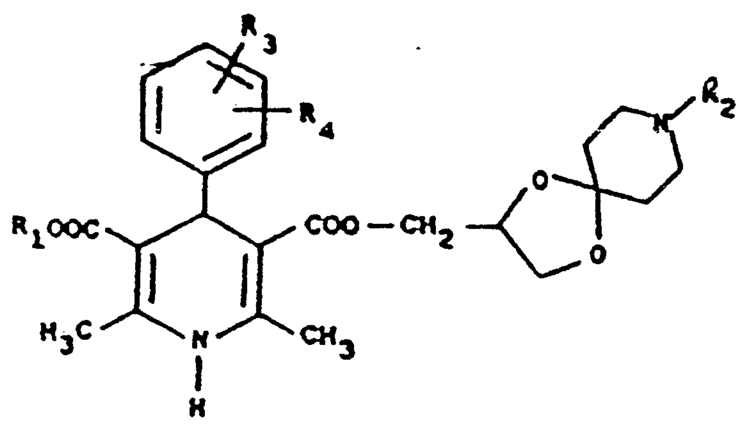

(I)

dans laquelle

$R_1$ représente un groupe alkyle en $C_1$ à $C_4$; $R_2$ représente un groupe alkyle en $C_1$-$C_4$, un groupe benzyle, un groupe benzoyle ou un groupe phényle éventuellement substitué par un ou plusieurs groupes alkoxy en $C_1$-$C_4$, alkyle en $C_1$-$C_4$, cyano, nitro, hydroxy, trifluorométhyle ou par un ou plusieurs atomes d'halogènes; $R_3$ et $R_4$, identiques ou différents, représentent chacun un atome d'hydrogène, un groupe nitro ou un atome de chlore.

(ii) leurs isomères optiques et diastéréoisomères; et,

(iii) les sels d'addition correspondants.

Parmi les groupes alkyle et alkoxy qui interviennent dans la définition des groupes $R_1$ et $R_2$ susvisés, on peut notamment mentionner les groupes $CH_3$, $CH_2CH_3$, $CH(CH_3)_2$, $C(CH_3)_3$, $OCH_3$, $OCH_2CH_3$, $OCH(CH_3)_2$ et $OC(CH_3)_3$.

Parmi les atomes d'halogène, qui conviennent selon l'invention, on peut citer les atomes de fluor, de chlore et de brome, l'atome d'halogène préféré étant l'atome de chlore.

Les groupes préférés que forment $R_3$ et $R_4$ avec le noyau phényl auquel ils sont liés sont le groupe 3-nitrophényle et le groupe 2,3-dichlorophényle.

Les composés de formule (1) selon l'invention peuvent être préparés selon deux méthodes :

La méthode A consiste à faire réagir un benzylidène-acéto-acétate de formule

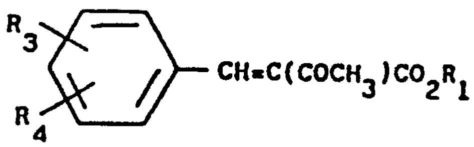

(II)

où $R_1$, $R_3$ et $R_4$ sont définis comme indiqué ci-dessus, avec un aminocrotonate de formule

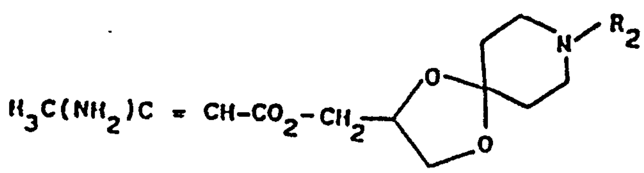

(III)

où $R_2$ est défini comme indiqué ci-dessus, selon la réaction dite de HANTZSCH.

De façon avantageuse, on fait réagir environ 1 mole de II avec environ 1 mole de III dans un solvant organique polaire (notamment un alkanol en $C_1$-$C_4$ tel que méthanol, éthanol, isopropanol, n-butanol, t-butanol), pendant 1 à 24 h, à une température comprise entre la température ambiante (15-20 °C) et la température de reflux du milieu réactionnel.

Les composés de formule II sont des substances connues qui peuvent être préparées selon la réaction dite de KNOEVENAGEL, voir à cet effet l'article récapitulatif de G. JONES, Organic Reactions 15, 204 (1975).

Les composés de formule III sont des produits nouveaux que l'on préconise de préparer comme suit.

1) réaction d'un alcool de formule

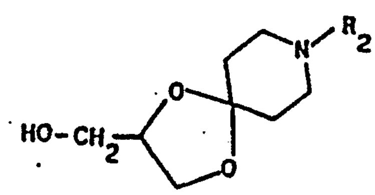

(IV)

où $R_2$ est défini comme indiqué ci-dessus, avec l'acide de Meldrum acétylé de formule

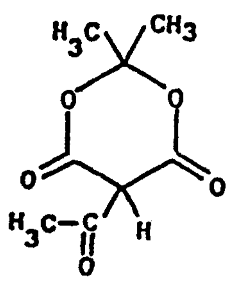

(V)

dans un solvant aromatique (notamment le benzène, le toluène, les xylènes) à une température comprise entre la température ambiante (15-20 °C) et la température de reflux du milieu réactionnel, pendant 0,2 à 4 h, pour obtenir un céto-ester nouveau de formule

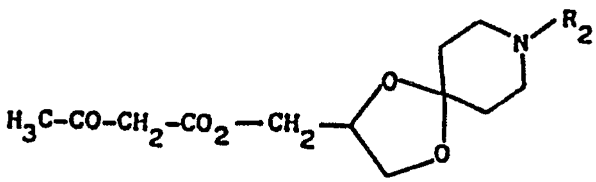

(VI)

où $R_2$ est défini comme indiqué ci-dessus
et,

2) traitement du dérivé de formule VI ainsi obtenu au moyen de $NH_3$ dans un solvant chloré (notamment $CH_2Cl_2$, $CHCl_3$) pendant 1 à 24 h.

Les composés de formules III et VI peuvent être utilisés sans avoir été préalablement purifiés. De façon avantageuse, on les utilise après les avoir purifiés soit par distillation (ou recristallisation) soit par "flash chromatography" (chromatographie sur colonne et sous pression) selon la technique décrite par W.C STILL et al. J. Org. Chem. 43. (N° 14), 2923 (1978).

Dans les tableaux I et II ci-après, on a consigné de façon non limitative les céto-esters de formule VI et respectivement les aminocrotonates de formule III, qui ont été préparés comme indiqué ci-dessus et qui interviennent dans la synthèse des composés selon l'invention.

Tableau I

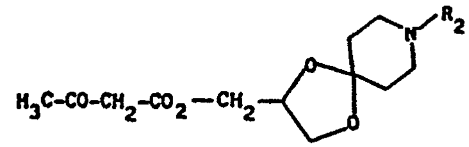

| $R_2$ | Temps de réaction (heures) | Solvant | Méthode de purification | rendement (% ) | Spectre de RMN (b) |
|---|---|---|---|---|---|
| $-CH_3$ | 1 | toluène | "flash chromatography toluène/isopropanol/ triéthylamine (95:5:5)v/v/v | 44 | 1,6-1,85 (4H,m); 2,27 (3H,s) ; 2,29(3H,s) ; 2,3-2,6 (4H,m); . 3,5(2H,s) ; 3,6-4,5 (5H,m) |
| 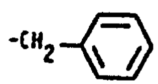 | 2 | toluène | "flash chromatography toluène/acétate d'é- thyle (6 :4) v/v | 71 | 1,6-1,9 (4H,m) ; 2,25 (3H,s) 2,3-2,7 (4H,s) ; 3,5(2H,s); 3,55(2H,s) ;3,6-4,4(5H,m) 7,2-7,4 (5H,m) (60 MHz) |
| 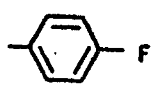 | 3 | toluène | (a) | 100 | 1,7-2 (4H,m) ; 2,27 (3H,s) ; 3,1-3,3 (4H,m) ;3,5 (2H,s) ; 3,6-4,5(5H,m) ;6,8-7 (4H,m) |

Tableau I ( suite 1)

| $R_2$ | Temps de réaction (heures) | Solvant | Méthode de purification | rendement (% ) | Spectre de RMN (b) |
|---|---|---|---|---|---|
| 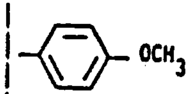 | 3 | toluène | (a) | 100 | 1,7-2 (4H,m) ; 2,27 (3H,s) ; 3,0-3,35 (4H,m) ; 3,5(2H,s) ; 3,76(3H,s) ; 4,0-4,5 (5H,m) ; 6,7-7 (4H,m) |
| 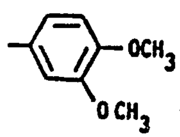 | 3 | toluène | (a) | 94 | 1,75-2 (4H,m) ; 2,27 (3H,s) ; 3,1-3,3 (4H,m) ; 3,5(2H,s) ; 3,83 (3H,s) ; 3,86 (3H,s) ; 3,7-4,5 (5H,m) ; 6,4-6,9(3H,m) |
| 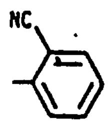 | 2 | toluène | "flash chromatography toluène/acétate d'éthyle (9:1) v/v | 70 | 1,8-2,1 (4H,m) ; 2,3 (3H,s) ; 3,1-3,4(4H,m) ; 3,5 (2H,s) ; 3,7-4,6(5H,m);6,8-7,6(4H,m)(60MHz) |

Tableau I ( suite 2)

| $R_2$ | Temps de réaction (heures) | Solvant | Méthode de purification | rendement (% ) | Spectre de RMN (b) |
|---|---|---|---|---|---|
| 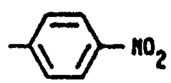 | 0,3 | benzène | chromatographie hexane/acétone (7 :3) v/v | 68 | 1,6-1,95 (4H,m) ; 2,27 (3H,s) ; 3,4-3,7 (m) et 3,5 (s)(6H) ; 3,75-4,5 (5H,m) ;6,8(2H,d) ; 8,1 (2H,d) |
| 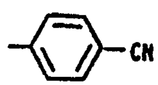 | 3 | toluène | (a) | 100 | 1,6-1,9(4H,m) ; 2,25 (3H,s) ; 3,3-3,6 (m) et 3,5 (s)(7H); 3,7-4,6 (5H,m) ; 6,85 (2H,d) 7,45 (2H,d) (60 MHz) |
| 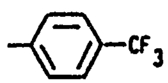 | 2 | toluène | (a) | 100 | 1,5-2(4H,m) ; 2,27 (3H,s) ; 3,3-4,6(m) et 3,5(s) (6H) ; 4-4,5 (5H,m) ; 6,8-6,95 (2H,d); 7,35-7,5 (2H,d) |

Tableau I ( fin )

| $R_2$ | Temps de réaction (heures) | Solvant | Méthode de purification | rendement (% ) | Spectre de RMN (b) |
|---|---|---|---|---|---|
| 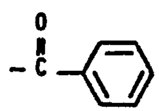 | 1 | Toluène | "flash chromatography" hexane/acétone (7 : 3 ) v/v | 94 | 1,6-1,9 (4H,m) ; 2,26 (3H,s) ; 3,4-4,4 (m) et 3,5 (s)(11H) ; 7,2-7,6 (5H,m) |
| 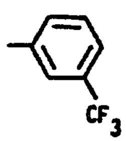 | 2 | toluène | (a) | 90 | 1,7-2,05 (4H,m) ; 2,28 (3H,s) ; 3,3-3,45 (4H,m) ; 3,5(1,8 H,s) 3,7-4,5(5H, m) ; 5,04 (0,1H,s) ; 6,9-7,5 (4H,m) ; 11,9 (0,1H, s) |

Notes: (a) sans purification
(b) spectre enregistré à 80 MHz, sauf indication contraire, dans $CDCl_3$, par rapport au TMS

Tableau II

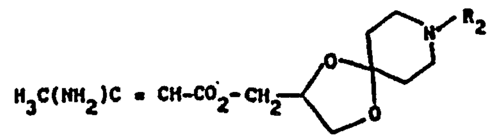

| $R_2$ | Temps de réaction (heures) | Solvant | Méthode de purification | rendement (% ) | Spectre de RMN |
|---|---|---|---|---|---|
| - $CH_3$ | 5 | méthanol | "flash chromatography" toluène/isopropanol/triéthylamine (95:5:5) v/v/v | 44 | 1,6-1,95 (m) et 1,9 (s) (7H) ; 2,3 (3H,s) ; 2,3-2,48 (4H,m) ; 3,6-4,4 (5H,m) ; 4,55 (1H,s) |
| 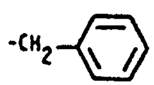 | 5 | chlorure de méthylène | (a) | 98 | 1,65-2 (m) + 1,9 (s) (7H) ; 2,4-2,75 (4H,m) ; 3,55 (2H,s) ; 3,6-4,5 (5H,m) ; 4,6 (1H,s) ; 7,25-7,4 (5H,m) |
| 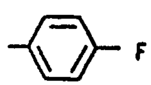 | 4 | chloroforme | (a) | 100 | |

Tableau II (suite 1)

| $R_2$ | Temps de réaction (heures) | Solvant | Méthode de purification | rendement (%) | Spectre de RMN (d) |
|---|---|---|---|---|---|
| 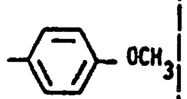 | 3 | chloro-forme | (a) | 100 | 1,7-2 (m) + 1,9 (s) (7H) ; 3,1-3,4 (4H,m) ; 3,76 (3H,s) ; 3,9-4,5 (5H,m) ;4,54 (1H,s) ; 6,7-7 (4H,m) |
| 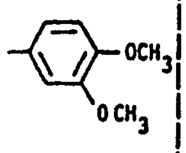 | 4 | chloro-forme | (a) | 100 | 1,75-2 (m) + 1,9(s) (7H) ; 3-3,3 (4H,m) ; 3,82 (3H,s) ; 3,86 (3H,s) ; 3,6-4,5 (5H,m) ; 4,55 (1H,s) ; 6,4-6,8 (3H,m) |
| 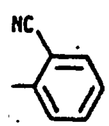 | 12 | chlorure de méthylène | (a) | 100 | 1,8-2,1(m)+1,9(s)(7H);3,1-3,4(4H,m) 3,7-4,45(5H,m);4,55(5H,m) ; 4,55 (1H,s);6,8-7,6(4H,m) (60 MHz) |

Tableau II ( suite 2)

| $R_2$ | Temps de réaction (heures) | Solvant | Méthode de purification | rendement (% ) | Spectre de RMN (d) |
|---|---|---|---|---|---|
| 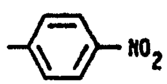 | 2 | chlorure de méthylène | (a) (b) | 97 | 1,6-1,95(m) + 1,9 (s) (7H); 3,4-3,7 (m,4H); 3,8-4,55(m,5H); 4,55 (1H,s) ;6,8 (2H,d);8,1(2H,d) |
| 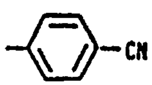 | 24 | chlorure de méthylène | (a) (c) | 77 | 1,6-1,9 (m) + 1,9(s) (7H) ; 3,3-3,6 (4H,m) ; 3,7-4,5(5H,m); 4,55 (1H,s) ;6,85 (2H,d) ; 7,45 (2H,d) (60 MHz) |
| 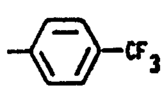 | 11 | chloro-forme | (a) | 100 | 1,7-2 (m) + 1,9 (s) (7H) ; 3,25-3,6 (4H,m) ; 3,7-4,4(5H,m); 4,56 (1H,s); 6,8-6,95 (2H,d) ; 7,35-7,5 (2H,d) |

Tableau II ( fin )

| $R_2$ | Temps de réaction (heures) | Solvant | Méthode de purification | rendement (% ) | Spectre de RMN (d) |
|---|---|---|---|---|---|
| 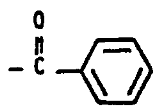 | 12 | Chlorure de méthylène | (a) | 100 (brut) | 1,55-1,95 (m) + 1,9(s)(7H) ; 3,4-4,4 (9H,m) ; 4,5 (1H,s) ; 7,2-7,6 (5H,m) |
| 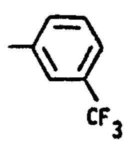 | 12 | Chloro-forme | (a) | 100 (brut) | 1,7-2 (m) + 1,9 (s)(7H) ; 3,2-3,5 (4H,m) ; 3,7-4,5(6H,m) ; 4,56 (1H,s) ; 6,9-7,5 (4H,m) |

Notes : (a)sans purification
(b)point de fusion : 176°C
(c)point de fusion : 170-175°C
(d)spectre enregistré à 80MHz, sauf indication contraire, dans $CDCl_3$ par rapport au TMS

La méthode B consiste en une cétalisation selon laquelle on fait réagir un diol, protégé ou non, de formule

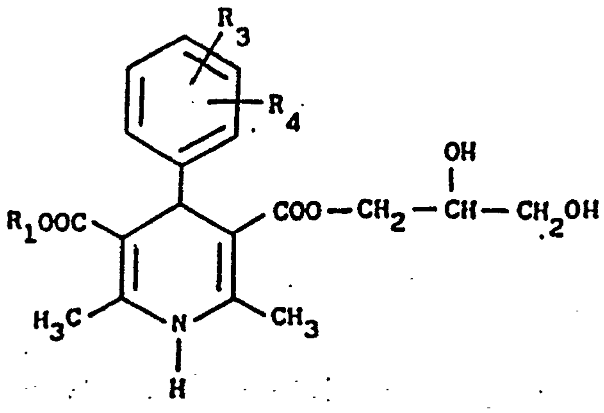

(VII)

où $R_1$, $R_3$ et $R_4$ sont définis comme indiqué ci-dessus, avec une pipéridinone de formule

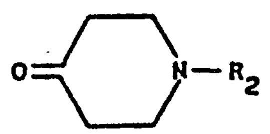

(VIII)

où $R_2$ est défini comme indiqué ci-dessus.

Le diol de formule VII peut être utilisé protégé, par exemple sous forme d'éthers méthylique ou éthylique substitués ou non, d'éther silylé, d'ester, de carbonate, d'acétal comme par exemple l'acétanilide, de cyclopentylidène, cyclohexylidène ou benzylidène et d'ortho-ester cyclique.

De façon avantageuse, on fait réagir environ 1 mole de VII avec environ 1 mole de VIII dans un solvant organique polaire, comme par exemple les alcools en $C_1$-$C_4$, les solvants aromatiques tels que le benzène, les xylènes, le toluène, ou leurs mélanges, en présence d'un acide minéral comme par exemple les acides sulfurique ou chlorohydrique ou d'un acide organique comme par exemple les acides paratoluène sulfonique ou benzènesulfonique, à une température comprise entre la température ambiante et la température de reflux du milieu réactionnel pendant 1 à 12 heures.

Le composé de formule VII peut être préparé selon une méthode connue en soi, notamment par ouverture du cycle dioxalannyle d'un composé de formule

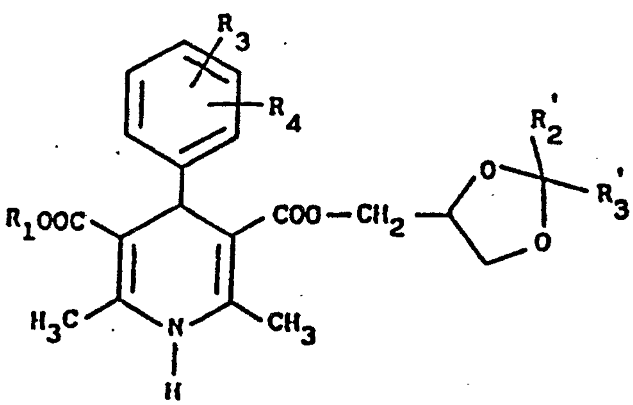

(IX)

dans laquelle $R_1$, $R_3$ et $R_4$ sont définis comme indiqué ci-dessus, et, $R'_2$ et $R'_3$ représentent chacun H ou un groupe alkyle en $C_1$-$C_4$, en milieu acide.

De façon non limitative, on a consigné dans le tableau III ci-après un certain nombre de composés de formule I selon l'invention.

TABLEAU III

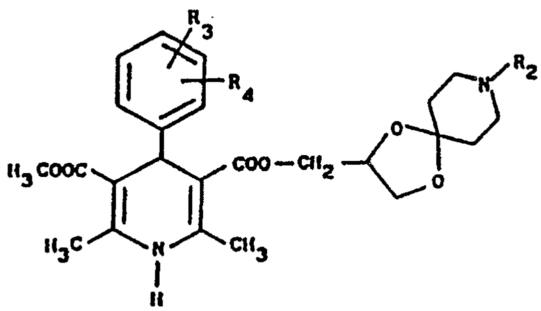

| Exemples | $R_2$ | $R_3$ | $R_4$ | F(°C) |
|---|---|---|---|---|
| 1 | 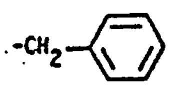 | 3-$NO_2$ | H | 87 |
| 2 | 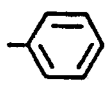 | 3-$NO_2$ | H | 89 |
| 3 | $-CH_3$ | 3-$NO_2$ | H | 62 à 76 (a) |
| 4 | 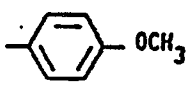 | 3-$NO_2$ | H | 65 à 85 (a) |
| 5 | 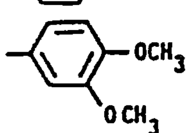 | 3-$NO_2$ | H | 65 à 85 (a) |
| 6 | 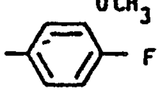 | 3-$NO_2$ | H | 70 à 95 (a) |
| 7 | 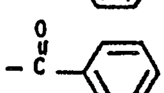 | 3-$NO_2$ | H | 95 à 115 (a) |
| 8 | 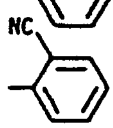 | 3-$NO_2$ | H | 70 à 93 (a) |

TABLEAU III (fin)

| Exemples | $R_2$ | $R_3$ | $R_4$ | F(°C) |
|---|---|---|---|---|
| 9 | 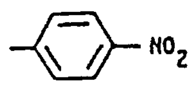 | $3\text{-}NO_2$ | H | 85 à 108 (a) |
| 10 | 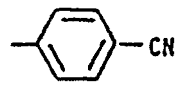 | $3\text{-}NO_2$ | H | 115 |
| 11 | 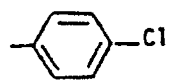 | $3\text{-}NO_2$ | H | 75 à 80 (a) |
| 12 | 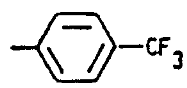 | $3\text{-}NO_2$ | H | 65 à 95 (a) |
| 13 | 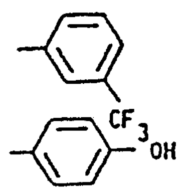 | $3\text{-}NO_2$ | H | 62 à 110 (a) |
| 14 | | $3\text{-}NO_2$ | H | 75 à 85 (a) |
| 15 | 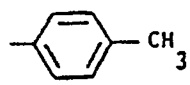 | $3\text{-}NO_2$ | H | 102 à 114 (a) |
| 16 | 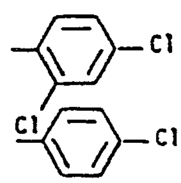 | $3\text{-}NO_2$ | H | 80 à 95 (a) |
| 21 | | 2-Cl | 3-Cl | 100 |

Notes : (a) Ces produits n'ont pas un point de fusion franc. la fusion s'étale sur la plage indiquée

(b) mousse

Les isomères énantiomères et diastéréoisomères peuvent être séparés des racémiques correspondants selon une méthode connue en soi, notamment par cristallisation fractionnée, par dédoublement ou autre.

Les énantiomères selon l'invention peuvent également être préparés selon un procédé caractérisé en ce que :

1) on fait réagir une dihydropyridine de formule

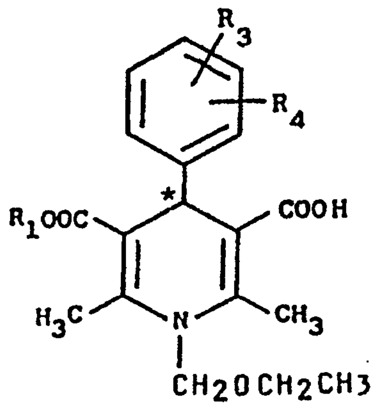

(X)

où $R_1$, $R_3$ et $R_4$ sont définis comme ci-dessus et le carbone noté * a la configuration R ou la configuration S, avec le carbonyldiimidazole, et,

2) sans isoler le dérivé intermédiaire formé, on soumet le milieu réactionnel à l'action d'un alcool de formule

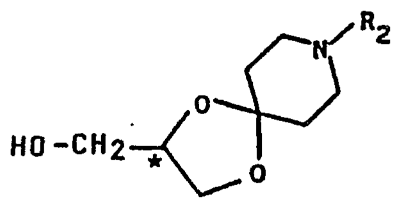

(IV')

où $R_2$ est défini comme ci-dessus et le carbone noté * a la configuration R ou la configuration S, pour obtenir un composé de formule

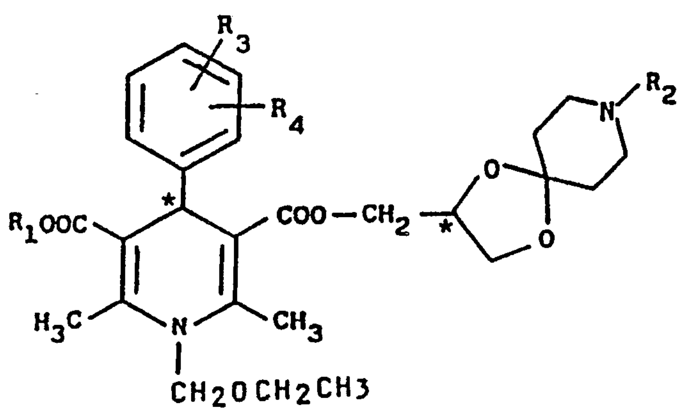

(XI)

où $R_1$, $R_2$, $R_3$ et $R_4$ sont définis comme ci-dessus et les carbones notés * ont une configuration R ou une configuration S, et,

3) on traite le composé de formule XI en milieu acide, pour obtenir un composé de formule

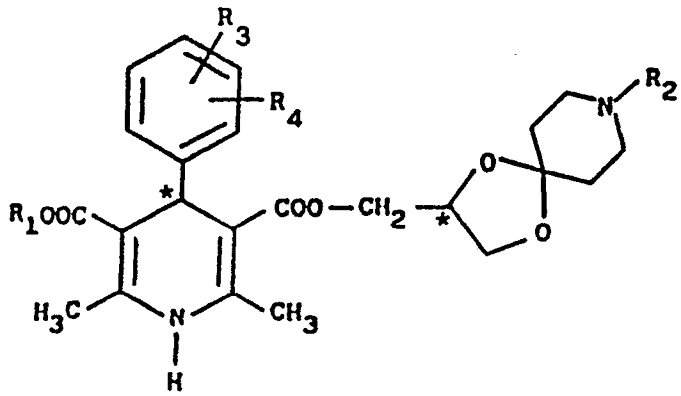

(I')

où $R_1$, $R_2$, $R_3$ et $R_4$ sont définis comme ci-dessus et les carbones notés * ont une configuration R ou une configuration S.

De façon avantageuse, on fait réagir environ 1 mole de X avec environ 1 mole de carbonyldiimidazole dans un solvant organique inerte (notamment la diméthylformamide, le diméthylacétamide ou le diméthylsulfoxyde), pendant 0,5 à 4 h, à une température comprise entre la température ambiante (15-20 °C) et environ 80 °C, puis on additionne environ 1 mole de IV' et environ 1 mole d'une base organique forte (notamment le 1,8-diazabicyclo [5.4.0] undec-7-ène, le 1,5-diazabicyclo [4.3.0] non-5-ène ou la 4-diméthylaminopyridine) et on chauffe le milieu réactionnel à une température comprise entre environ 40 °C et environ 150 °C, pendant 1 à 10 h. On isole de façon classique le composé de formule XI que l'on traite en milieu acide fort (notamment l'acide chlorhydrique N) dans un solvant organique (notamment les cétones) pour obtenir un composé optiquement actif de formule I'.

Les composés de formule I peuvent être salifiés selon les méthodes classiques connues de l'homme de l'art. On préférera notamment les sels d'addition obtenus par salification par les acides forts biologiquement acceptables, comme par exemple les acides chlorhydrique, bromhydrique, méthanesulfonique, nitrique ou sulfurique.

Selon l'invention, on préconise une composition thérapeutique qui est caractérisée en ce qu'elle renferme, en association avec un excipient physiologiquement acceptable, au moins un composé choisi parmi l'ensemble constitué par les dérivés de formule I ci-dessus, leurs isomères énantiomères et leurs diastéréoisomères, et leurs sels d'addition.

Bien entendu, dans une telle composition, le principe actif intervient en quantité pharmaceutiquement efficace.

Selon l'invention, on préconise également l'utilisation d'une substance choisie parmi (i) les dérivés de formule I, (ii) leurs isomères énantiomères et leurs diastéréoisomères, et (iii) leurs sels d'addition non toxiques, pour l'obtention d'un médicament antihypertenseur destiné à une utilisation en thérapeutique humaine vis-à-vis de l'hypertension et des insuffisances cardiaques.

D'autres avantages et caractéristiques de l'invention seront mieux compris à la lecture qui va suivre d'exemples de préparation et de résultats d'essais pharmacologiques. L'ensemble de ces éléments n'est pas limitatif mais donné à titre d'illustration. Dans lesdits exemples, les configurations R et S sont désignées conformément aux règles de nomenclature des Chemical Abstracts.

## PREPARATION I

Obtention du 3-oxo-butanoate de (8-aza-1,4-dioxa-8-phényl-spiro [4,5] décane-2-yl)méthyle

On chauffe au reflux pendant 3 heures un mélange de 19 g ($7{,}66.10^{-2}$ mole) de (8-aza-1,4-dioxa-8-phényl-spiro [4,5] décane-2-yl)méthanol, 14,2 g ($7{,}66.10^{-2}$ mole) d'acide de meldrum acétylé (composé de formule V) et 200 ml de toluène. Le solvant est évaporé et le produit brut obtenu est purifié par "flash chromatography" en éluant avec un mélange toluène/acétate d'éthyle (19 : 1) v/v puis hexane / acétone (19 : 1) v/v. On obtient 19,8 g (rendement : 78 %) du produit attendu sous forme d'une huile jaune.

Spectre de RMN : 80 MHz TMS ($CDCl_3$)

1,7-2 (4H,m) ; 2,26 (3H,s) ; 3,2-3,45 (4H,m) ; 3,5 (2H,s) ; 3,6-4,5 (5H,m) ; 6,7-7,3 (5H,m)

PREPARATION II

Obtention de l'aminocrotonate de (8-aza-1,4-dioxa-8-phényl-spiro [4,5] décane-2-yl)méthyle

On dissout 19,8 g ($5{,}96.10^{-2}$mole) du produit obtenu à la préparation I dans 200 ml de méthanol. On fait barbotter de l'ammoniac gazeux séché sur potasse pendant 2 heures dans la solution maintenue à 0 ° C, puis pendant 4 heures à température ambiante. Le solvant est évaporé et le produit brut obtenu est purifié par "flash chromatography" en éluant avec un mélange hexane/acétone (19 : 1) v/v puis hexane/acétone (9 : 1) v/v. On obtient 15 g (rendement : 75 %) du produit attendu sous forme d'une huile jaune pâle.

Spectre de RMN : 80 MHz TMS ($CDCl_3$)

1,7-2(m) et 1,89(s) (7H) ; 3,2-3,45 (4H,m) ; 3,5-4,5 (5H,m) ; 4,55(1H,s) ; 6,7-7 (3H,m) ; 7,2-7,3 (2H,m)

PREPARATION III

Obtention du 1,4-dihydro-2,6-diméthyl-4-(3-nitrophényl)-pyridine-3,5-dicarboxylate de méthyle et de (8-aza-1,4-dioxa-8-phényl-spiro [4,5] décane-2-yl)méthyle

Exemple 2

On chauffe à reflux pendant 4 heures un mélange de 14,7 g ($4{,}44.10^{-2}$mole) du produit obtenu à la préparation II et de 11 g ($4{,}44.10^{-2}$mole) de 3-nitrobenzylidène-acétylacétate de méthyle et 300 ml de tertiobutanol. Le solvant est évaporé et le produit brut est purifié par "flash chromatography" en éluant avec un mélange hexane/acétone (19 : 1) v/v puis toluène/isopropanol (100 : 1) v/v puis hexane/acétone (8 : 2) v/v. On obtient 5,7 g (rendement : 25 %) d'huille jaune qui se solidifie sous forme d'une mousse amorphe fondant à 89 ° C.

Spectre de RMN 80 MHz TMS ($CDCl_3$)

1,8 (4H,m) ; 2,36 (6H, s) ; 3,3 (4H,m) ; 3,5-4,3 (8H,m); 5,11 (1H,s) ; 5,8 (1H,s) ; 6,8-8,1 (9H,m)

PREPARATION IV

Obtention du 1,4-dihydro-2,6-diméthyl-4-(3-nitrophényl)-pyridine-3,5-dicarboxylate de méthyle et de (8-aza-1,4-dioxa-8-phényl-spiro [4,5] décane-2-yl)méthyle

Exemple 2

On dissout 2,03 g ($0{,}5.10^{-2}$mole) de 1,4-dihydro-2,6-diméthyl-4-(3-nitrophényl)-pyridine-3,5-dicarboxylate de méthyle et de 1,2-dihydroxypropane-3-yle dans 10 ml de méthanol, puis on additionne 0,96 g ($0{,}55.10^{-2}$mole) de N-phényl-4-pipéridinone, 20 ml de toluène et 0,3 g d'acide paratoluène-sulfonique. Le mélange est chauffé 3 heures à reflux, puis ramené à température ambiante et hydrolysé sur 100 ml d'une solution aqueuse saturée de bicarbonate de soude. Le milieu réactionnel est ensuite extrait trois fois avec des portions de 30 ml d'acétate d'éthyle. Les phases organiques obtenues sont rassemblées et lavées trois fois avec des portions de 20 ml d'eau. La solution organique obtenue est séchée sur sulfate de magnésium, filtrée et le solvant est évaporé. L'huile obtenue est purifiée par "flash chromatography" en éluant avec un mélange toluène/isopropanol (95 : 5) v/v. On obtient 15,5 g (rendement : 55 %) du produit attendu sous

forme d'une mousse jaune dont les caractéristiques sont identiques à celles du produit obtenu à la préparation III.

PREPARATION V

Obtention du 1,4-dihydro-2,6-diméthyl-4-(3-nitrophényl)-pyridine-3,5-dicarboxylate de méthyle et (8-aza-8-(4-chlorophényl)-1,4-dioxa-spiro [4,5] décane-2-yl)méthyle

Exemple 11

On dissout 2,03 g ($0,5.10^{-2}$mole) de 1,4-dihydro-2,6-diméthyl-4-(3-nitrophényl)-pyridine-3,5-dicarboxylate de méthyle et de 1,2-dihydroxy-3-propyle dans 10 ml de méthanol, puis on ajoute 1,15 g ($0,55.10^{-2}$mole) de N-(4-chlorophényl)-4-pipéridinone, 20 ml de toluène et 0,2 g d'acide paratoluène-sulfonique. Le mélange est chauffé 3 heures à reflux puis refroidi à température ambiante et hydrolysé sur 100 ml de solution aqueuse saturée de bicarbonate de soude. Le milieu réactionnel est extrait trois fois avec des portions de 30 ml d'acétate d'éthyle. Les phases organiques sont reassemblées et lavées trois fois avec des portions de 20 ml d'eau puis séchées sur sulfate de magnésium, filtrées et le solvant est évaporé. L'huile obtenue est purifiée par "flash chromatography" en éluant avec un mélange toluène/isopropranol (95 : 5) v/v. On obtient 1,7 g (rendement : 57 %) du produit attendu sous forme d'une poudre cristallisée qui fond à 145° - 150° C.

Spectre de RMN : 80 MHz TMS ($CDCl_3$)

1,8 (4H,m) ; 2,36-2,38 (6H,2s) ; 3,26 (4H,m) ; 3,5-4,4 (m) dont 3,64 (s) et 3,65 (s) (8H) ; 5,12 (1H,s) ; 5,85-(1H,s) ; 6,6-8,1 (8H,m).

PREPARATION VI

Obtention du 3-oxobutanoate de (8-aza-8-(4-chlorophényl)-1,4-dioxa-spiro [4,5] décane-2-yl)méthyle

On chauffe à reflux pendant une heure un mélange de 3,5 g (0,0122 mole) de (8-aza-8-(4-chlorophényl)-1,4-dioxa-spiro [4,5]décane-2-yl)méthanol, 2,95 g (0,0159 mole) de l'acide de Meldrum acétylé et 100 ml de toluène. Le solvant est ensuite évaporé et l'huile obtenue est purifiée par "flash chromatography" en éluant avec un mélange toluène/acétate d'éthyle (8 : 2) v/v. On obtient 3,5 g (rendement : 78 %) de solide jaune fondant à 59° C.

Spectre de RMN : 80 MHz TMS ($CDCl_3$)

1,65-2 (4H,m) ; 2,27 (3H,s) ; 3,1-3,4 (4H,m) ; 3,5 (2H,s) ; 3,6-4,5(5H,m) ; 6,83 (2H,d) ; 7,2 (2H,d)

PREPARATION VII

Obtention de l'aminocrotonate de (8-aza-8-(4-chlorophényl)-1,4-dioxa-spiro [4,5] décane-2-yl)méthyle

On fait barbotter à température ambiante pendant 6 h de l'ammoniac séché sur potasse dans un mélange de 3,5 g ($0,95.10^{-2}$ mole) de 3-oxobutanoate de (8-aza-8-(4-chlorophényl)-1,4-dioxa-spiro [4,5] décane-2-yl)méthyle, 100 ml de chlorure de méthylène sec et 5 g de tamis moléculaire 4 Å, puis on laisse le milieu réactionnel sous agitation pendant 12 heures. Le tamis moléculaire est filtré et le solvant évaporé. On obtient 3,06 g de produit jaune cristallisé fondant à 60° C (Rendement : 88 %).

Spectre de RMN : 80 MHz TMS ($CDCl_3$)

1,7-2(m) et 1,91 (s) (7H) ; 3,1-3,4(4H,m) ; 3,6-4,5 (5H,m); 4,56 (1H,s) ; 6,75-6,90 (2H,d) ; 7,1-7,25 (2H,d)

PREPARATION VIII

Obtention du 1,4-dihydro-2,6-diméthyl-4-(3-nitrophényl)pyridine-3,5-dicarboxylate de méthyle et de (8-aza-8-(4-chlorophényl)-1,4-dioxa-spiro [4,5] décane-2 yl)méthyle

Exemple 11

On chauffe à reflux pendant huit heures un mélange de 3,1 g ($0,85.10^{-2}$mole) d'aminocrotonate de (8-aza-8-(4-chlorophényl)-1,4-dioxa-spiro [4,5] décane-2-yl)méthyle, 2,1 g ($0,85.10^{-2}$mole) de 3-nitrobenzylidène-acétyl-acétate de méthyle et 100 ml de tertiobutanol. Le solvant est évaporé et le produit brut obtenu est purifié par "flash chromatography" en éluant avec un mélange toluène/isopropanol (95 : 5) v/v puis avec un mélange hexane/acétone (8 : 2) v/v. On obtient 1,7 g (rendement : 35 %) du produit attendu dont les caractéristiques physiques sont identiques à celles du produit obtenu à la préparation V.

PREPARATION IX

Obtention du 1,4-dihydro-2,6-diméthyl-4-(3-nitrophényl)-pyridine-3,5-dicarboxylate de méthyle et de (8-aza-1,4-dioxa-8-phényl-spiro [4,5] décane-2-yl) méthyle

Exemple 2

On chauffe à reflux pendant 6 h un mélange de 0,22g ($5.10^{-4}$ mole) de 1,4-dihydro-2,6-diméthyl-4-(3-nitrophényl)-pyridine-3,5-dicarboxylate de méthyle et de (2,2-diméthyl-1,3-dioxolanne-4-yl)méthyle, 0,26g ($1,5.10^{-5}$ mole) de N-phényl-4-pipéridinone, 8 ml de toluène et 35 mg d'acide paratoluène sulfonique. Le mélange réactionnel est hydrolysé sur une solution aqueuse saturée de bicarbonate de soude. Le milieu obtenu est extrait à l'acétate d'éthyle. Les phases organiques obtenues sont lavées à l'eau, séchées sur sulfate de magnésium, filtrées et le solvant est évaporé. Le produit brut obtenu est purifié par "flash chromatography" en éluant avec un mélange toluène/isopropranol (98 : 2) v/v. On obtient 0,07 g (rendement : 25 %) du produit attendu dont les caractéristiques physiques sont identiques à celles du produit obtenu à la préparation III.

PREPARATION X

Obtention du 1,4-dihydro-2,6-diméthyl-4-(3-nitrophényl)-pyridine-3,5-dicarboxylate de méthyle et de (8-aza-1,4-dioxa-8-(4-hydroxyphényl)-spiro [4,5] décane-2-yl)méthyle

Exemple 14

Selon le mode opératoire décrit dans la préparation IV à partir de 6 g (0,015 mole) de 1,4-dihydro-2,6-diméthyl-4-(3-nitrophényl)-pyridine-3,5-dicarboxylate de méthyle et de 1,2-dihydroxypropane-3-yle, de 1,9 g (0.01 mole) de N-(4-hydroxyphényl)-4-pipéridinone et de 0,5 g d'acide paratoluène-sulfonique dans 30 ml de butanol et 60 ml de toluène à reflux pendant 11 h, on obtient, après deux "flash chromatography" successives en éluant avec un mélange hexane/acétone (1 : 1) v/v, 2,2 g (rendement : 38 %) du produit attendu sous forme de mousse.
F = 102 à 114°C

Spectre de RMN : 80 MHz TMS ($CDCl_3$)

1,7-2 (4H,m) ; 2,37 (6H,s) ; 3,0-3,3 (4H,m) ; 3,63 et 3,64 (3H,ds) ; 3,6-4,5 (5H,m) ; 5,12 (1H,s) ; 6,08 (1H,s) ; 6,70 et 6,88 (4H,dd) ; 7,25-8,15 (4H,m)

PREPARATION XI

Obtention du 1,4-dihydro-2,6-diméthyl-4-(3-nitrophényl)-pyridine-3,5-dicarboxylate de méthyle et de (8-aza-1,4-dioxa-8-(4-méthylphényl)-spiro[4,5] décane-2-yl) méthyle

Exemple 15

Selon le mode opératoire décrit dans la préparation IV, à partir de 2 g (0,005 mole) de 1,4-dihydro-2,6-diméthyl-4-(3-nitrophényl)-pyridine-3,5-dicarboxylate de méthyle et de dihydroxy-1,2-propane-3-yle et de 1,86 g (0,01 mole) de N-(4-méthylphényl)-4-pipéridinone et de 0,2 g d'acide paratoluène-sulfonique dans 20 ml de toluène et 10 ml de butanol à reflux pendant 24 heures, on obtient après deux "flash chromatography" successives en éluant avec un mélange hexane/acétone (6 : 4) v/v puis de l'éther, 2,1 g (rendement : 70 %) du produit attendu sous forme de mousse fondant sur une plage de 75 à 85° C.

Spectre de RMN : 80 MHz TMS ($CDCl_3$)

1,7-1,95 (4H,m) ; 2,26 (3H,s) ; 2,35 (6H,s) ; 3.05-3,3 (4H,m) ; 3,63 (3H,s) ; 3,6-4,5 (5H,m) ; 5,12 (1H,s) ; 6,17 (1H,s) ; 6,8 et 7,07 (4H,dd) ; 7,2-8,15 (4H,m)

PREPARATION XII

Obtention du 3-oxo-butanoate de (8-aza-1,4-dioxa-8-(4-trifluorométhylphényl)-spiro [4,5] décane-2-yl)-méthyle

On chauffe à reflux pendant deux heures un mélange de 4,75 g (0,015 mole) de (8-aza-1,4-dioxa-8-(4-trifluorométhylphényl)-spiro [4,5] décane-2-yl)méthanol, 3 g (0,016 mole) d'acide de Meldrum acétylé (composé de formule V) et 50 ml de toluène. Le mélange est ramené à température ambiante et on additionne de l'eau. La phase organique est décantée puis lavée à l'eau jusqu'à pH neutre et séchée. On obtient 6 g d'huile.

Spectre de RMN : 80 MHz TMS ($CDCl_3$)

1,5-2 (4H,m) ; 2,27 (3H,s) ; 3,3-4,6 (m) + 3,5(s) (6H) ; 4-4,5 (5H,m) ; 6,8-6,95 (2H,d) ; 7,35-7,5 (2H,d)

PREPARATION XIII

Obtention de l'aminocrotonate de (8-aza-1,4-dioxa-8-(4-trifluorométhylphényl)-spiro [4,5] décane-2-yl)-méthyle.

On dissout 6 g (0,015 mole) du produit obtenu à la préparation XII dans 100 ml de chloroforme. On fait barbotter de l'ammoniac gazeux pendant 3 heures dans la solution, puis pendant 8 heures après avoir ajouté du tamis moléculaire dans le milieu réactionnel. Le mélange est filtré, la phase organique obtenue est évaporée. On obtient 6 g de solide fondant à 122° C.

Spectre de RMN : 80 MHz TMS ($CDCl_3$)

1,7-2 (m) + 1,9 (s) (7H) ; 3,25-3,6 (4H,m) ; 3,7 - 4,4 (5H,m) ; 4,56 (1H,s) ; 6,8 - 6,95 (2H,d) ; 7,35-7,5 (2H,d)

PREPARATION XIV

Obtention du 1,4-dihydro-2,6-diméthyl-4-(3-nitrophényl)-pyridine 3,5-dicarboxylate de méthyle et de (8-aza-1,4-dioxa-8-(4-trifluorométhylphényl)-spiro [4,5] décane-2-yl)méthyle.

Un mélange de 6 g (0,015 mole) du produit obtenu à la préparation XIII, et de 3,7 g (0,015 mole) de 3-nitrobenzylidène-acétyl-acétate de méthyle dans le tertiobutanol est porté à reflux pendant 1 heure. Le solvant est évaporé et le produit brut est purifié par deux "flash chromatography" successives en éluant avec un mélange toluène/isopropanol (9 : 1) v/v puis toluène/isopropranol (19 : 1) v/v. On obtient 2,3 g (rendement : 25 %) d'huile qui se solidifie sous forme d'une mousse amorphe fondant sur une plage de 65 à 95 ° C.

Spectre de RMN : 80 MHz TMS ($CDCl_3$)

1,8 (4H,m) ; 2,36-2,38 (6H,ds) ; 3,4 (4H,m) ; 3,5-4,5(m) + 3,64 (s) + 3,65 (s) (8H) ; 5,12 (1H,s) ; 5,76 (1H,s) ; 6,7-8,1(8H,m)

PREPARATION XV

Obtention du 1,4-dihydro-2,6-diméthyl-4-(3-nitrophényl)-pyridine-3,5-dicarboxylate de méthyle et de (8-aza-8-(2,4-dichlorophényl)-1,4-dioxa-spiro [4,5] décane-2-yl)méthyle,

Exemple 16

Selon le mode opératoire décrit à la préparation IV au départ de 4,06 g ($10^{-2}$ mole) de 1,4-dihydro-2,6-diméthyl-4-(3-nitrophényl)-pyridine-3,5-dicarboxylate de méthyle et de 1,2-dihydroxypropane-3-yleet de 2,44 g ($10^{-2}$ mole) de N-(2,4-dichlorophényl)-4-pipéridinone, on obtient 5,6 g (rendement : 90 %) du produit attendu fondant sur une plage de 80 à 95 ° C.

Spectre de RMN : 80 MHz TMS ($CDCl_3$)

1,87 (4H,m) ; 2,39 (6H,s) ; 3,05 (4H,m) ; 3,5-4,4 (m) et 3,64 (s) (8H) ; 5,12 (1H,s) ; 5,99 (1H,s) ; 6,95-8,1 (7H,m)

PREPARATION XVI

Obtention du 1,4-dihydro-2,6-diméthyl-4-(3-nitrophényl)-pyridine-3,5-dicarboxylate de méthyle et de 1,2-dihydroxypropane-3-yle. (Produit intermédiaire intervenant dans la préparation IV ci-dessus).

Dans un ballon de 250 ml, on introduit 11,1 g ($2,48.10^{-2}$ mole) de 1,4-dihydro-2,6-diméthyl-4-(3-nitrophényl)-pyridine-3,5-dicarboxylate de méthyle et de (2,2-diméthyl-1,3-dioxolanne-4-yl)-méthyle, 50 ml de méthanol et 50 ml d'acide HCl 1N. On agite le milieu réactionnel pendant 1 h à 15-20 ° C puis on neutralise avec $NaHCO_3$. On évapore le méthanol et extrait avec 100 ml d'acétate d'éthyle. On lave la phase acétate d'éthyle à l'eau jusqu'à pH neutre, puis sèche (sur $MgSO_4$), filtre et évapore le filtrat sous pression réduite. On recueille le résidu d'évaporation (9,2 g ; rendement : 91 %) qui est essentiellement constitué par le 1,4-dihydro-2,6-diméthyl-4-(3-nitrophényl)-pyridine-3,5-dicarboxylate de méthyle et de 1,2-dihydroxypropane-3-yle, produit se présentant sous la forme d'une mousse jaune fondant à 90 ° C.

Spectre de RMN : 80 MHz TMS ($CDCl_3$)

1,73 (1H,s) ; 2,37 (7H,s) ; 3,5 (2H,m) ;
3,6 (3H,s) ; 3,8 (1H,m) ; 4,19 (2H,m) ;
5,1 (1H,s) ; 6,01 (1H,s) ; 7,3-8,03 (4H,m)

PREPARATION XVII

Obtention du (R)-8-aza-1,4-dioxa-8-(4-chlorophényl)-2-(2-propènyloxyméthyl)-spiro [4,5] décane

On chauffe à reflux un mélange de 16 g (0,075 mole) de N-(4-chlorophényl)-4-pipéridinone, 1,6 g d'acide paratoluène sulfonique et 70 ml de méthanol anhydre. Lorsque le cétal est formé, on ajoute 10 g (0,075 mole) de (S)-3-(2-propènyloxy)-1,2-propanediol. On distille alors le méthanol en le remplaçant par du toluène. Après une heure de réaction, on laisse refroidir à température ambiante. Le mélange réactionnel est hydrolysé sur une solution aqueuse saturée de bicarbonate de soude. Le milieu obtenu est extrait à l'acétate d'éthyle. Les phases organiques sont lavées à l'eau, séchées sur sulfate de magnésium, filtrées et le solvant est évaporé. Le produit brut obtenu est purifié par "flash chromatography" en éluant avec un mélange hexane/acétone (9 : 1) v/v. On obtient 16 g (rendement : 75 %) du produit pur dont les caractéristiques physiques sont les suivantes :

Spectre RMN : 80 MHz TMS ($CDCl_3$)

1,7 - 2 (4H,m) ; 3,1 - 3,4 (4H,m) ; 3,5 - 4,5 (7H,m) ; 5,05 - 5,45 (2H,m) ; 5,75 - 6,2 (1H,m) ; 6,83 (2H,d) ; 7,2 (2H,d).

Pouvoir rotatoire : C = 0,65 g/100 ml (méthanol)
$[\alpha]_D^{20}$ = - 8,2

Point de fusion : 40° C

PREPARATION XVIII

Obtention du (S)-8-aza-1,4-dioxa-8-(4-chlorphényl)-2-(2-propènyloxyméthyl)-spiro [4,5] décane

De façon analogue à la préparation précédente, par réaction du (R)-3-(2-propènyloxy)-1,2-propanediol sur le cétal, on obtient le produit attendu dont le spectre de RMN et le point de fusion sont identiques à ceux du produit obtenu à la préparation précédente.

Pouvoir rotatoire : C = 0,65 g/100 ml (méthanol)
$[\alpha]_D^{20}$. = + 10,3

PREPARATION XIX

Obtention du (R)-8-aza-2-hydroxyméthyl-1,4-dioxa-8-(4-chlorophényl)-spiro [4,5] décane

On dissout 20,1 g (0,062 mole) du produit obtenu à la préparation XVII dans un mélange de 450 ml d'acétone et 45 ml d'eau. On ajoute goutte à goutte à température ambiante un mélange de 15,7 g d'oxyde mercurique et de 18,5 g de chlorure mercurique dans 110 ml d'un mélange acétone/eau (10:1) v/v. Le milieu réactionnel est agité pendant une heure puis filtré sur Célite[R] et le solvant est évaporé. Le résidu est ensuite repris à l'éther, puis lavé avec une solution aqueuse d'iodure de potassium. L'éther est évaporé. Le produit brut obtenu est purifié par "flash chromatography" en éluant avec un mélange toluène/acétate d'éthyle (8 : 2) v/v. On obtient 16,3 g (rendement : 93 %) du produit attendu sous forme de cristaux blancs.

Pouvoir rotatoire : C = 0,46 g/100 ml (méthanol)
$[\alpha]_D^{20}$ = - 4,0

Point de fusion : 125° C

PREPARATION XX

Obtention du (S)-8-aza-2-hydroxyméthyl-1,4-dioxa-8-(4-chlorophényl)-spiro [4,5] décane

De façon analogue à la préparation précédente, au départ du produit obtenu à la préparation XVIII, on obtient le produit attendu dont les caractéristiques physiques sont les suivantes :

Pouvoir rotatoire : C = 0,46 g/100 ml (méthanol)

$[\alpha]_D^{20}$ = + 4,9

Point de fusion : 130 °C

PREPARATION XXI

Obtention du N-(éthoxyméthyl)-1,4-dihydro-2,6-diméthyl-4-(3-nitrophényl)-pyridine-3,5-dicarboxylate de méthyle et de (8-aza-8-(4-chlorophényl)-1,4-dioxa-spiro [4,5] décane-2-yl)méthyle (R-(R*, S*))

On dissout 2,925 g (0,0075 mole ) d'acide (R)-N-(éthoxyméthyl)-1,4-dihydro-2,6-diméthyl-4-(3-nitrophényl)-pyridine-3-méthoxycarbonyl-5-carboxylique et 1,34 g (0,0082 mole) de carbonyldiimidazole dans 50 ml de diméthylformamide. Le mélange est chauffé à 40 °C pendant une heure. On ajoute alors au milieu réactionnel 2,13 g (0,0075 mole) du produit obtenu à la préparation XX et 1,12 ml (0,0075 mole) de 1,8-diazabicyclo [5.4.0] undec-7-ène. Le mélange est porté à 100 °C.Après 2 heures, on laisse refroidir, puis le mélange est versé dans 100 ml d'eau. Le milieu est extrait 3 fois avec 30 ml d'acétate d'éthyle. Les phases organiques obtenues sont lavées deux fois avec 10 ml d'eau, séchées sur sulfate de magnésium, filtrées et le solvant est évaporé. Le produit brut obtenu est purifié par "flash chromatography" en éluant avec un mélange toluène/isopropanol (97:3) v/v. On obtient 4,8 g (rendement : 98 %) d'une huile.

Spectre de RMN : 80 MHz TMS ($CDCl_3$)

1,23 (3H,t) ; 1,65 - 2 (4H,m) ; 2,56 (6H,s) ; 3,05 - 3,35 (4H,m) ; 3,47 (2H,q) ; 3,69 (3H,s,s) ; 3,50 - 4,40 (5H,m) ; 4,86 (2H,s) ; 5,19 (1H,s) ; 6,83 (2H,d) ; 7,19 (2H,d) ; 7,25 - 7,75 (2H,m) ; 7,8 - 8,1 (2H,m)

PREPARATION XXII

Obtention du 1,4-dihydro-2,6-diméthyl-4-(3-nitrophényl)-pyridine-3,5-dicarboxylate de méthyle et de (8-aza-8-(4-chlorophényl)-1,4-dioxa-spiro [4,5] décane-2-yl)méthyle (R-(R*,S*)).

Exemple 17

On dissout 4,9 g (0,0075 mole) du produit obtenu à la préparation XXI dans 120 ml d'acétone, 25 ml d'acide chlorhydrique N et 10 ml d'eau. On agite le mélange à température ambiante pendant 8 heures. Le milieu réactionnel est versé sur 200 ml d'une solution aqueuse de bicarbonate de soude. On extrait 3 fois avec 50 ml d'acétate d'éthyle. Les phases organiques sont lavées deux fois avec 20 ml d'eau séchées sur sulfate de magnésium et le solvant est évaporé. Le produit brut obtenu est purifié sur colonne de chromatographie en éluant à l'éther. On obtient 3 g (rendement : 67 %) du produit attendu fondant à 171 °C.

Spectre de RMN : 500 MHz TMS ($CDCl_3$)

1,82 (4H,m) ; 2,38 (6H,d) ; 3,26 (4H,m) ; 3,66 (3H,s) ; 3,73 ( 1H,m) ; 4,05 (1H,m ) ; 4,10 (2H,m) ; 4,32 (1H,m) ; 5,11 (1H,s) ; 5,87 (1H,s) ; 6,85 (2H,m) ; 7,19 (2H,m) ; 7,37 (1H,m) ; 7,66 (1H,m) ; 8,00 (1H,m) ; 8,10 (1H,m)

Pouvoir rotatoire : C = 0,5 g/100 ml (méthanol)

$[\alpha]_D^{20}$ = + 29,2

PREPARATION XXIII

Obtention du 1,4-dihydro-2,6-diméthyl-4-(3-nitrophényl)-pyridine-3,5-dicarboxylate de méthyle et de (8-aza-8-(4-chlorophényl)-1,4-dioxa-spiro [4,5] décane-2-yl)méthyle (S-(R*,R*)).

Exemple 18

De façon analogue aux préparations XXI et XXII, au départ d'acide (R)-N-(éthoxyméthyl)-1,4-dihydro-2,6-diméthyl-4-(3-nitrophényl)-pyridine-3-méthoxycarbonyl -5-carboxylique et du produit obtenu à la préparation XIX on obtient le produit attendu fondant à 175° C.

Spectre de RMN : 500 MHz TMS (CDCl3)

1,80 (4H,m) ; 2,37 ( 6H,d) ; 3,25 (4H,m) ; 3,58 (1H,m) ; 3,64 (3H,s) ; 3,97 (1H,m) ; 4,10 (2H,m) ; 4,33 (1H,m) ; 5,10 (1H,s) ; 5,83 (1H,s) ; 6,85 (2H,d) ; 7,20 (2H,d) ; 7,38 (1H,m) ; 7,65 (1H,m) ; 8,00 (1H,m) ; 8,10 (1H,m)

Pouvoir rotatoire : C = 0,46 g/100 ml (méthanol)

$[\alpha]_D^{20}$ = + 14,6

PREPARATION XXIV

Obtention du 1,4-dihydro-2,6-diméthyl-4-(3-nitrophényl)-pyridine-3,5-dicarboxylate de méthyle et de (8-aza-8-(4-chlorophényl)-1,4-dioxa-spiro [4,5] décane-2-yl)méthyle (S-(R*,S*)).

Exemple 19

De façon analogue aux préparations XXI et XXII, au départ d'acide (S)-N-(éthoxyméthyl)-1,4-dihydro-2,6-diméthyl-4-(3-nitrophényl)-pyridine-3-méthoxycarbonyl-5-carboxylique et du produit obtenu à la préparation XIX, on obtient le produit attendu fondant à 151-154° C.

Spectre de RMN : 500 MHz TMS ($CDCl_3$)

1,81 (4H,m) ; 2,38 (6H,d) ; 3,26 (4H,m) ; 3,66 (3H,s) ; 3,73 (1H,m) ; 4,05 (1H,m) ; 4,10 (1H,m) ; 4,32 (1H,m) ; 5,11 (1H,s) ; 5,86 (1H,s) ; 6,84 (2H,m) ; 7,19 (2H,m) ; 7,37 (1H,m) ; 7,66 (1H,m) ; 7,99 (1H,m) ; 8,10 (1H,m).

Pouvoir rotatoire : C = 0,5 g/100 ml (méthanol)

$[\alpha]_D^{20}$ = - 23,0

PREPARATION XXV

Obtention du 1,4-dihydro-2,6-diméthyl-4-(3-nitrophényl)-pyridine-3,5-dicarboxylate de méthyle et de (8-aza-8-(4-chlorophényl)-1,4-dioxa-spiro [4,5] décane-2-yl)méthyle (R-(R*,R*)).

Exemple 20

De façon analogue aux préparations XXI et XXII, au départ d'acide (S)-N-(éthoxyméthyl)-1,4-dihydro-2,6-diméthyl-4-(3-nitrophényl)-pyridine-3-méthoxycarbonyl-5-carboxylique et du produit obtenu à la préparation XX, on obtient le produit attendu fondant à 176-179° C.

Spectre de RMN : 500 MHz TMS ($CDCl_3$)

1,80 (4H,m) ; 2,37 (6H,d) ; 3,25 (4H,m) ; 3,59 (1H,m) ; 3,65 (3H,s) ; 3,97 (1H,m) ; 4,12 (2H,m) ; 4,33 (1H,m) ;

5,10 (1H,s) ; 5,87 (1H,s) ; 6,84 (2H,m) ; 7,17 (2H,m) ; 7,38 (1H,m) ; 8,00 (1H,m) ; 8,10 (1H,m).

Pouvoir rotatoire : C = 0,4 g/100 ml (méthanol)

$[\alpha]_D^{20}$ = -10,9

PREPARATION XXVI

Obtention du 1,4-dihydro-2,6-diméthyl-4-(2,3-dichlorophényl)-pyridine-3,5-dicarboxylate de méthyle et de (8-aza-8-(4-chlorophényl)-1,4-dioxa-spiro [4,5] décane-2-yl)méthyle

Exemple 21

Selon le mode opératoire décrit dans la préparation VIII à partir de 2,73 g (0,01 mole) de 2,3-dichlorobenzylidène-acétyl-acétate de méthyle, de 3,66 g (0,01 mole) d'aminocrotonate de (8-aza-8-(4-chlorophényl)-1,4-dioxa-spiro [4,5] décane-2-yl)méthyle et de 30 ml de tertio-butanol à reflux pendant une heure, on obtient, après une première "flash chromatography" en éluant avec un mélange éther/hexane (1 : 1) v/v, puis une deuxième "flash chromatography" en éluant à l'éther et après recristallisation dans l'éthanol, 2,6 g (rendement : 42 %) du produit attendu fondant à 100 °C.

Spectre de RMN : 80 MHz TMS ($CDCl_3$)

1,77 (4H,m) ; 2,31 (6H,d) ; 3,25 (4H,m) ; 3,40 - 4,40 (5H,m) ; 3,60 (3H,s) ; 5,44 (1H,s) ; 5,74 (1H,s) ; 6,80 (2H,d) ; 7,13 (2H,d) ; 7,00 - 7,40 (3H,m).

Dans le tableau IV ci-après, on a regroupé un certain nombre de produits obtenus selon le mode opératoire décrit dans la préparation III. Le solvant utilisé est le tertiobutanol et la température de réaction est la température de reflux du milieu réactionnel. On a indiqué la masse des produits mis en réaction, la durée de la réaction, la méthode de purification utilisée, la masse de produit de formule 1 selon l'invention obtenue, le rendement de la réaction ainsi que le spectre de RMN du produit attendu.

Tableau IV

| Ex. | amino-crotonate III (g) | nitrobenzylidène acéto acétate II (g) | durée (heures) | méthode de purification | masse obtenue (g) | Rdt (%) | spectre de RMN |
|---|---|---|---|---|---|---|---|
| 1 | 2,5 | 1,8 | 2,5 | 4"flash-chromatography"<br>Toluène/acétate d'éthyle (8 : 7) v/v<br>Toluène/isopropanol (19 : 1) v/v<br>Toluène/isopropanol (9 : 1) v/v<br>Toluène/acétate d'éthyle/triéthylamine (80:5:5) v/v/v | 2,1 | 50 | 1,7(4H,m); 2,5 (m) + 2,36(s)(10H) ;<br>3-4,3 (m)+ 3,6(s) + 3,5 (s) (10H) ;<br>5,1 (1H,s) ; 6,0(1H,s)<br>7,2-8,08 (9H, m) |
| 3 | 5,2 | 4,8 | 4 | 2"flash chromatography"<br>chloroforme/méthanol (50 : 1) v/v<br>toluène/isopropanol/triéthylamine (190:10:1) v/v/v | 3,9 | 40 | 1,76 (4H,m) ; 2,49 (m) + 2,34 (s) + 2,3 (s)(13H) ;<br>3,65 (3H,s) ;<br>3,5-4,4(5H,m) ;<br>5,11(1H,s) ; 6,06(1H,s) ;<br>7,2-8,08 (4H,m) |
| 4 | 6,4 | 4,4 | 1 | "flash chromatography"<br>hexane/acétone (7: 3) v/v | 6 | 56 | 1,83(4H,m) ; 2,36(6H,s) ;<br>3,16(4H,m) ; 3,63(3H,s) ;<br>3,64 (3H,s); 3,7 (3H,s) ;<br>3,5-4,4(5H,m) ;5,12(1H;s);<br>5,89(1H,s) ;6,7-8,1(8H,m) |

Tableau IV (suite 1)

| Ex. | amino-crotonate III (g) | nitrobenzylidène acéto acétate II (g) | durée (heures) | méthode de purification | masse obtenue (g) | Rdt (%) | spectre de RMN |
|---|---|---|---|---|---|---|---|
| 5 | 7 | 4,4 | 1 | "flash chromatography"<br>hexane/acétone (7 : 3) v/v | 2,5 | 22 | 1,85(4H,m) ;2,36 et 2,38 (6H,ds) ; 3,17 (4H,m) ; 3,5-4,4 (m)+ 3,64(s) + 3,83(s) + 3,87(s) (14H); 5,12 (1H,s) ; 5,85(1H,s); 6,4-8,1 (7H,m) |
| 6 | 9 | 6,5 | 1 | 2"flash chromatography"<br>hexane/acétone (7 : 3) v/v éther | 2,5 | 18 | 1,8 (4H,m) ; 2,36 et 2,38 (6H,ds) ; 3,2 (4H,m) ; 3,5-4,5 (m) + 3,60 et 3,65 (ds) (8H) ; 5,11 (1H,s) ; 5,9 (1H,s) ; 6,8-8,1 (8H,m) |
| 7 | 15,4 | 10,5 | 12 | 3"flash chromatography"<br>hexane/acétone (7 : 3) v/v<br>toluène/isopropanol (20 : 1) v/v<br>hexane/acétone (6 : 4) v/v | 3,3 | 13 | 1,66(4H,m) ;2,33 et 2,35 (6H,ds) ; 3,5-4,2 (m) + 3,6 (s) (12H) ; 5,1 (1H,s) ; 6,2 (1H,s) ; 7,3-8,1 (9H,m) |

Tableau IV ( suite 2)

| Ex. | amino-crotonate III (g) | nitrobenzylidène acéto acétate II (g) | durée (heures) | méthode de purification | masse obtenue (g) | Rdt (%) | spectre de RMN |
|---|---|---|---|---|---|---|---|
| 8 | 10,46 | 7,2 | 10 | 4"flash chromatography" toluène/isopropanol (98 : 2) v/v toluène/isopropanol (97 : 3) v/v toluène/isopropanol (97 : 3) v/v éther/acétone(1 : 1)v/v | 3,7 | 22 | 1,9 (4H,m) ; 2,37 et 2,39 (6H,ds) ;3,28(4H,m); 3,5-4,5(m) + 3,64(ds)(8H) 5,19 (1H,s) ; 5,95 (1H,s) 7-8,1 (8H,m) |
| 9 | 6 | 3,96 | 4 | 3 "flash chromatography" toluène/isopropanol (98 : 2 ) v/v toluène/isopropanol (98 : 2 ) v/v éther/acétone (1 : 1) v/v | 4,1 | 42 | 1,78 (4H,m) ; 2,36 et 2,39 (6H,ds) ; 3,2-4,2 (12H,m) ; 5,12 (1H,s); 6,07 (1H,s) ; 6,6-8,14 (8H,m) |
| 10 | 3,1 | 2,5 | 3 | 2 "flash chromatography" toluène/isopropanol (98 : 2) v/v éther | 3,1 | 60 | 1,77(4H,m) ;2,36 et 2,38 (6H,ds) ; 3,2-4,4(m) 3,64 et 3,65(ds) (12H) ; 5,11(1H,s) ; 6,05(1H,s) ; 6,6-8,1 (8H,m) |

Tableau IV ( fin )

| Ex. | amino-crotonate III (g) | nitrobenzylidène acéto acétate II (g) | durée (heures) | méthode de purification | masse obtenue (g) | Rdt (%) | spectre de RMN |
|---|---|---|---|---|---|---|---|
| 12 | 6 | 3,7 | 1 | 2 " flash chromatography" toluène/isopropanol (9 : 1) v/v toluène/ isopropanol (9 : 1) v/v | 2,3 | 25 | 1,8 (4H,m) ; 2,36 et 2,38 (6H,ds) ; 3,4(4H,m); 3,5-4,5 (m) + 3,64 et 3,65 (ds) (8H) ; 5,12 (1H,s) ; 5,76 (1H,s) 6,7- 8,1 (8H,m) |
| 13 | 4,4 | 2,5 | 1 | 2 "flash chromatography" toluène/acétate d'éthyle (10 : 1) v/v hexane/acétone (3 : 1) v/v | 1,5 | 24 | 1,5-1,95 (4H,m) ; 2,37 (6H,s) ; 3,1-3,45 (m,4H); 3,5- 4,4 (m) + 3,64 (s) (8H) ; 5,13 (1H,s) ; 6,04 (1H,s) ; 7-8,15 (8 H,m) |

L'activité antihypertensive des produits selon l'invention a été mise en évidence par administration orale à des rats spontanément hypertendus âgés de 16 à 20 semaines. La pression artérielle systolique est mesurée par pléthysmographie et la fréquence cardiaque est obtenue à partir du tracé de pression. Les composés étudiés sont administrés par voie orale. Les effets antihypertenseurs maximum obtenus sont consignés dans le tableau V ci-après. Sauf indication contraire, on a utilisé des lots de 6 animaux. La

signification statistique de l'effet à la 24ème heure est donnée entre parenthèse où * signifie $p < 0,05$, ** $p < 0,01$, *** $p < 0,001$ selon une analyse de variance suivie d'un test t de Student.

Les produits selon l'invention, ainsi que la nicardipine prise comme référence, ont été également testés par administration orale chez le chien hypertendu périnéphritique. Des chiens bâtards de 20 à 25 kg ou beagle de 10 à 15 kg sont rendus hypertendus par enveloppement des deux reins dans une feuille de cellophane selon la technique décrite par I.H. Page (J.A.M.A., vol. 113, N° 23, pp 2046-48, 1939). Dix à douze semaines après l'intervention et après conditionnement des animaux aux situations de mesure, la pression artérielle systolique et la fréquence cardiaque sont mesurées à l'aide d'un capteur piézo-électrique et d'un manchon gonflable (appareillage Apelex-BP recorder). Les produits sont administrés par voie orale à la dose de 3 mg/kg et les paramètres sont mesurés 0,5, 1, 2, 4, 6, 8 et 24 heures après la prise.

A titre d'exemple, on a observé une baisse de la pression artérielle systolique de (44,3 ± 4,8) % pour le composé de l'exemple 11, la durée de l'effet étant supérieure à 24 heures. La nicardipine entraîne une chute de pression artérielle systolique de (25,8 ± 17,6) %, la durée de l'effet n'étant pas supérieure à 6 heures.

TABLEAU V

Activité antihypertensive chez le rat spontanément hypertendu (voie orale)

| Produit | Dose (mg/kg) | % de variation de la pression systolique ± écart standard | Durée de l'effet |
|---|---|---|---|
| Ex 1 | 30 | - 54,1 ± 5,2 | 8 à 24 |
| Ex 2 | 10 | - 44,3 ± 14,8 | > 24 (*) |
| Ex 2 | 30 | - 51,7 ± 5,8 | > 24 (***) |
| Ex 3 | 30 | - 36,4 ± 13,3 | 8 à 24 |
| Ex 4 | 30 | - 37 ± 5 | > 24 (***) |
| Ex 5 | 30 | - 43,6 ± 6,1 | > 24 (*) |
| Ex 6 | 1(a) | - 19,1 ± 10,4 | 8 à 24 |
| Ex 6 | 3 | - 29,5 ± 8,0 | 8 à 24 |
| Ex 6 | 10(b) | - 50,1 ± 3,4 | > 24 (***) |
| Ex 6 | 30 | - 52,6 ± 6,4 | > 24 (***) |
| Ex 7 | 30 | - 43,7 ± 6,6 | > 24 (*) |
| Ex 8 | 30 | - 19,7 ± 10,8 | > 24 (*) |
| Ex 9 | 30 | - 30,8 ± 7,7 | > 24 (**) |
| Ex 10 | 30 | - 36,3 ± 4,5 | 8 à 24 |
| Ex 11 | 3 | - 36,3 ± 11 | > 24 (***) |
| Ex 11 | 10 | - 48,0 ± 11,1 | > 24 (***) |
| Ex 11 | 30 | - 57,4 ± 6,0 | > 24 (***) |
| Ex 12 | 10 | - 47,8 ± 4,6 | > 24 (***) |

TABLEAU V (fin)

| Produit | Dose (mg/kg) | % de variation de la pression systolique ± écart standard | Durée de l'effet |
|---|---|---|---|
| Ex 13 | 3 | - 29,3 ± 9,1 | > 24 (*) |
| Ex 13 | 10 | - 43,5 ± 7,0 | > 24 (***) |
| Ex 14 | 10 | - 23,3 ± 12,2 | > 24 (*) |
| Ex 15 | 10 | - 36,0 ± 11,3 | > 24 (*) |
| Ex 16 | 3 | - 45,5 ± 7,0 | > 24 (*) |
| Ex 17 | 3 | - 41,7 ± 2,7 | 8 à 24 |
| Ex 17 | 10 | - 46,6 ± 4,3 | > 24 (***) |
| Ex 18 | 3 | - 29,7 ± 13,0 | 8 à 24 |
| Ex 18 | 10 | - 44,7 ± 3,1 | > 24 (***) |
| Ex 19 | 3 | - 13,4 ± 7,7 | 4 |
| Ex 19 | 10 | - 32,3 ± 9,0 | 8 à 24 |
| Ex 20 | 3 | - 9,0 ± 9,6 | 6 |
| Ex 20 | 10 | - 21,3 ± 9,4 | 8 à 24 |
| Ex 21 | 3 | - 27,7 ± 10,6 | > 24 (***) |
| Ex 21 | 10 | - 41,0 ± 9,3 | > 24 (**) |
| nicardipine | 30 | - 46,2 ± 6,8 | 8 à 24 |
| nicardipine | 10 | - 43,7 ± 4,7 | 4 à 6 |

Notes : (a) : moyenne de 5 animaux

(b) : moyenne de 4 animaux

## Revendications

**1.** Dérivé ester hétérocyclique dissymétrique de l'acide 1,4-dihydropyridine-3,5-dicarboxylique, caractérisé en ce qu'il est choisi parmi l'ensemble comprenant

(i) les esters répondant à la formule générale

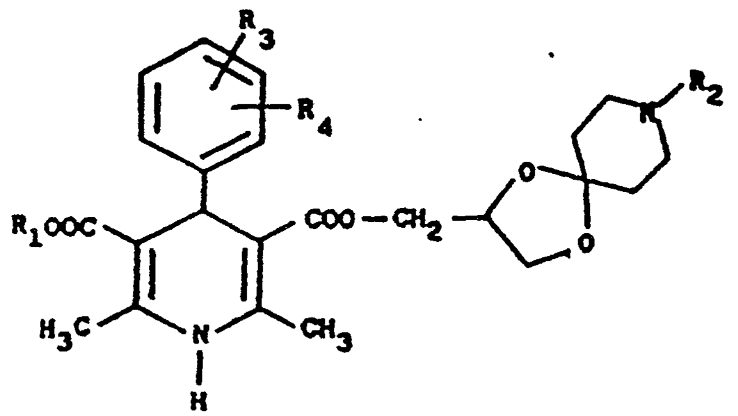

(I)

dans laquelle

$R_1$ représente un groupe alkyle en $C_1$-$C_4$;

$R_2$ représente un groupe alkyle en $C_1$-$C_4$, un groupe benzyle, un groupe benzoyle ou un groupe phényle éventuellement substitué par un ou plusieurs groupe alcoxy en $C_1$-$C_4$, alkyle en $C_1$-$C_4$,cyano, nitro, hydroxy, trifluorométhyle ou par un ou plusieurs atomes d'halogène ; $R_3$ et $R_4$, identiques ou différents, représentent chacun un atome d'hydrogène, un groupe nitro ou un atome de chlore;

(ii) leurs isomères optiques et diastéréoisomères; et,

(iii) les sels d'addition correspondants.

2. Dérivé selon la revendication 1, caractérisé en ce que $R_2$ est $CH_3$, $CH_2C_6H_5$, $COC_6H_5$, $C_6H_5$, 4-méthoxyphényle, 3,4-diméthoxyphényle, 4-fluorophényle, 2-cyanophényle, 4-cyanophényle, 4-nitrophényle, 3-trifluorométhylphényle, 4-trifluorométhylphényle,4-chlorophényle, 2,4-dichlorophényle, 4-hydroxyphényle, 4-méthylphényle.

3. Dérivé selon la revendication 1, caractérisé en ce que $R_3$ et $R_4$ forment avec le noyau phényl auquel ils sont liés le groupe 3-nitrophényle ou le groupe 2,3-dichlorophényle.

4. Dérivé selon la revendication 1, caractérisé en ce qu'il s'agit du 1,4-dihydro-2,6-diméthyl-4-(3-nitrophényl)-pyridine-3,5-dicarboxylate de méthyle et de (8-aza-8-(4-chlorophényl)-1,4-dioxa-spiro [4,5] décane-2-yl) méthyle.

5. Dérivé selon la revendication 1, caractérisé en ce qu'il s'agit du 1,4-dihydro-2,6-diméthyl-4-(3-nitrophényl)-pyridine-3,5-dicarboxylate de méthyle et de (8-aza-8-(4-chlorophényl)-1,4-dioxa-spiro [4,5] décane-2-yl) méthyle (R-($R^*$,$S^*$)).

6. Dérivé selon la revendication 1, caractérisé en ce qu'il s'agit du 1,4-dihydro-2,6-diméthyl-4-(3-nitrophényl)-pyridine-3,5-dicarboxylate de méthyle et de (8-aza-8-(4-chlorophényl)-1,4-dioxa-spiro [4,5] décane-2-yl) méthyle (S-($R^*$,$R^*$)).

7. Dérivé selon la revendication 1, caractérisé en ce qu'il s'agit du 1,4-dihydro-2,6-diméthyl-4-(2,3-dichlorophenyl)-pyridine-3,5-dicarboxylate de méthyle et de (8-aza-8-(4-chlorophényl)-1,4-dioxa-spiro [4,5] décane-2-yl) méthyle.

8. Dérivé selon la revendication 1, caractérisé en ce qu'il s'agit du 1,4-dihydro-2,6-diméthyl-4-(3-nitrophényl)-pyridine-3,5-dicarboxylate de méthyle et de (8-aza-8-(2,4-dichlorophényl)-1,4-dioxa-spiro [4,5] décane-2-yl) méthyle

9. Composition thérapeutique caractérisée en ce qu'elle renferme en association avec un excipient physiologiquement acceptable au moins un composé choisi parmi l'ensemble constitué par les dérivés de formule I selon la revendication 1, leurs isomères énantiomères et leurs diastéréoisomères, et les sels d'addition correspondants.

**10.** Utilisation d'une substance choisie parmi (i) les composés de formule I selon la revendication 1, (ii) leurs isomères énantiomères et leurs diastéréoisomères et iii) les sels d'addition correspondants non-toxiques, pour l'obtention d'un médicament antihypertenseur destiné à une utilisation en thérapeutique humaine vis-à-vis de l'hypertension et des insuffisances cardiaques.

**11.** Procédé de préparation d'un composé de formule I selon la revendication 1, caractérisé en ce que l'on fait réagir un benzylidène-acéto-acétate de formule

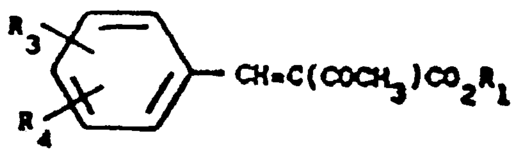

(II)

où $R_1$, $R_3$ et $R_4$ sont définis comme indiqué dans la revendication 1, avec un aminocrotonate de formule

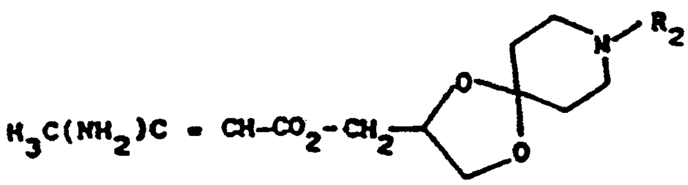

(III)

où $R_2$ est défini comme indiqué dans la revendication 1, par mise en contact d'environ 1 mole de II avec environ 1 mole de III dans un solvant organique polaire, notamment un alcanol en $C_1$-$C_4$, pendant 1 à 24 h, à une température comprise entre la température ambiante et la température de reflux du milieu réactionnel,
le composé III étant notamment obtenu selon le mécanisme suivant :
1°) réaction d'un alcool de formule

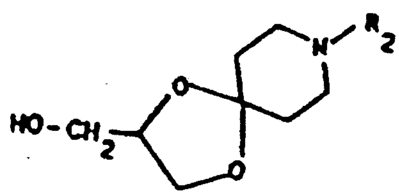

(IV)

où $R_2$ est défini comme indiqué dans la revendication 1, avec l'acide de Meldrum acétylé de formule

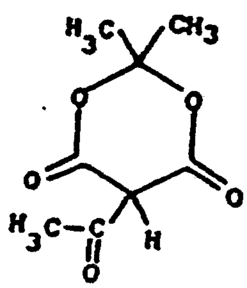

(V)

dans un solvant aromatique (notamment le benzène, le toluène, les xylènes) à une température

comprise entre la température ambiante (15-20 ° C) et la température de reflux du milieu réactionnel, pendant 0,2 à 4 h, pour obtenir un céto-ester nouveau de formule

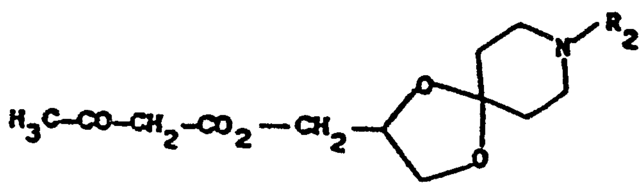

(VI)

où $R_2$ est défini comme indiqué dans la revendication 1, et

2°) traitement du dérivé de formule VI ainsi obtenu au moyen de $NH_3$ dans un solvant chloré (notamment $CH_2Cl_2$, $CHCl_3$) pendant 1 à 24 h.

**12.** Procédé de préparation d'un composé de formule I selon la revendication 1, caractérisé en ce que l'on fait réagir un diol de formule

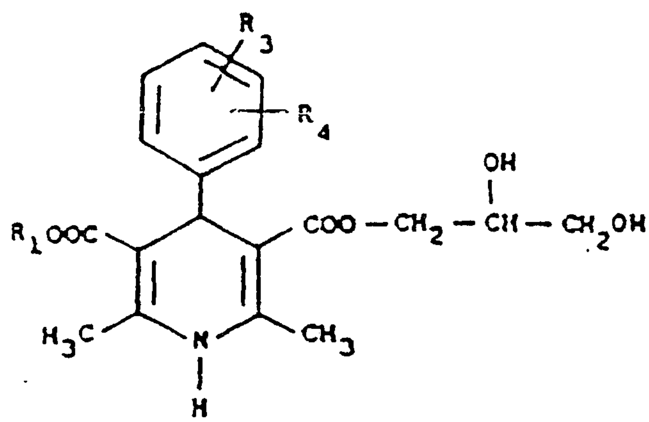

(VII)

où $R_1$, $R_3$ et $R_4$ sont définis comme indiqué dans la revendication 1, avec une pipéridinone de formule

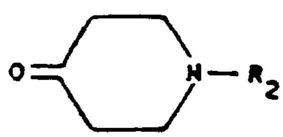

(VIII)

où $R_2$ est défini comme indiqué dans la revendication 1, les fonctions OH dudit diol VII pouvant le cas échéant être protégées lors de la cétalisation effectuée à raison d'environ 1 mole de VII avec environ 1 mole de VIII, dans un solvant polaire en présence d'un acide minéral ou organique, à température comprise entre la température ambiante et la température de reflux du milieu réactionnel, pendant 1 à 12 h, ledit diol VII étant notamment obtenu par ouverture du cycle dioxolannyle d'un composé de formule

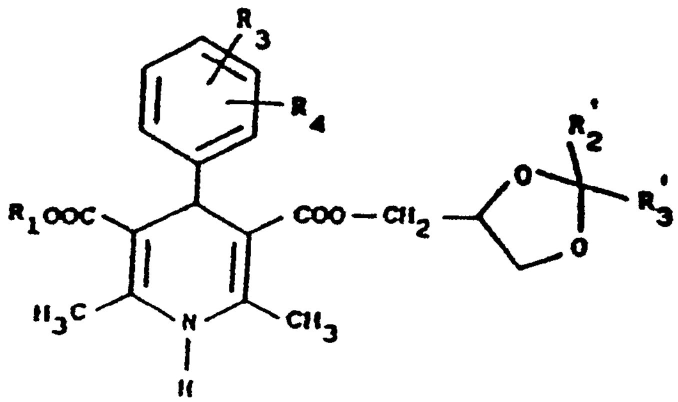

(IX)

où $R_1$, $R_3$ et $R_4$ sont définis comme indiqué dans la revendication 1, et $R'_2$ et $R'_3$ représentent chacun H ou un groupe alkyle en $C_1$-$C_4$, en milieu acide.

**13.** Procédé de préparation d'un composé de formule I selon la revendication 1, caractérisé en ce que :
1°) on fait réagir environ une mole d'une dihydropyridine de formule

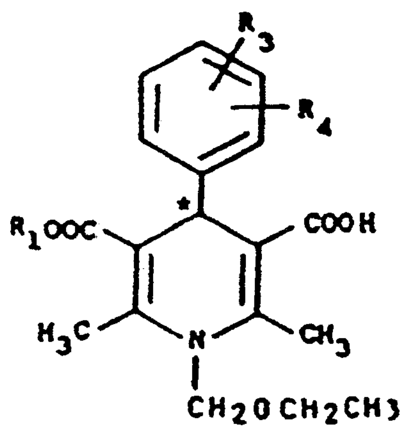

(X)

où $R_1$, $R_3$ et $R_4$ sont définis comme indiqué dans la revendication 1, et le carbone noté * a la configuration R ou la configuration S, avec environ une mole de carbonyldiimidazole, dans un solvant organique inerte, pendant 0,5 à 4 h, à une température comprise entre la température ambiante et environ 80° C;
2°) sans isoler le dérivé intermédiaire formé, on soumet le milieu réactionnel à l'action d'environ une mole d'un alcool de formule

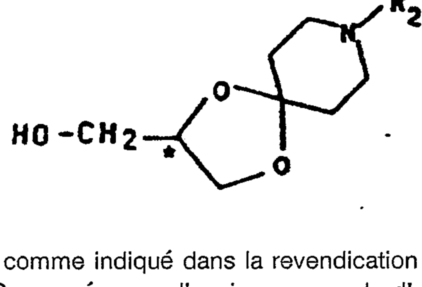

(IV')

où $R_2$, est défini comme indiqué dans la revendication 1 et le carbone noté * a la configuration R ou la configuration S, en présence d'environ une mole d'une base organique forte, pendant 1 à 10 h, à une température comprise entre environ 40° C et environ 150° C pour obtenir un composé de formule

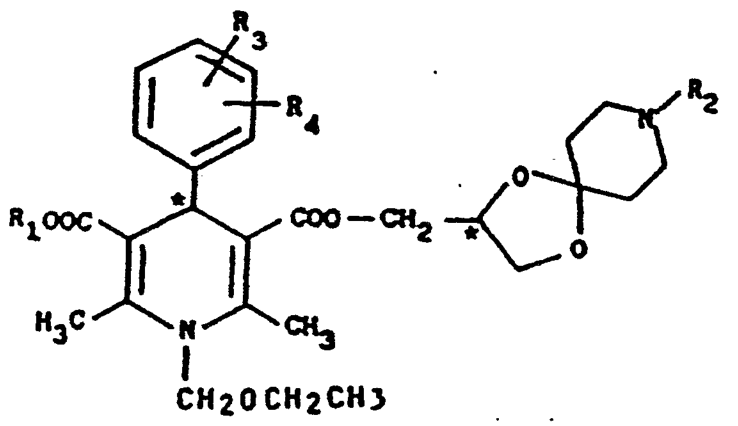

(XI)

où $R_1$, $R_2$, $R_3$ et $R_4$ sont définis comme indiqué dans la revendication 1, et les carbones notés * ont une configuration R ou une configuration S, et,

3°) on traite le composé de formule XI en milieu acide fort dans un solvant organique, pour obtenir un composé de formule

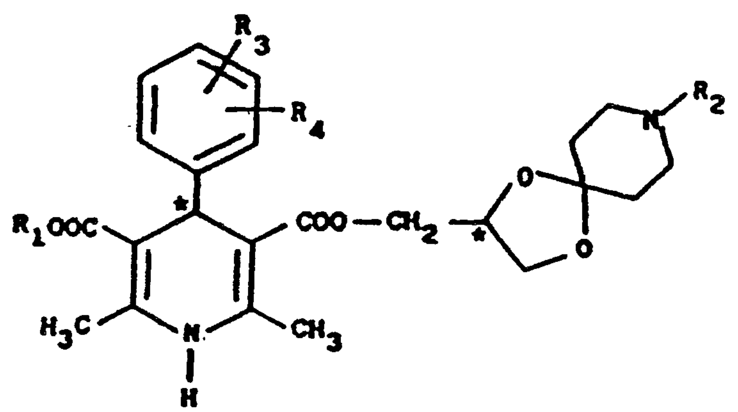

(I')

où $R_1$, $R_2$, $R_3$ et $R_4$ sont définis comme indiqué dans la revendication 1, et les carbones notés * ont une configuration R ou une configuration S.

**14.** Produit intermédiaire intervenant dans la synthèse des composés de formule I selon la revendication 1, caractérisé en ce qu'il est choisi parmi l'ensemble constitué par

a) les aminocrotonates de formule

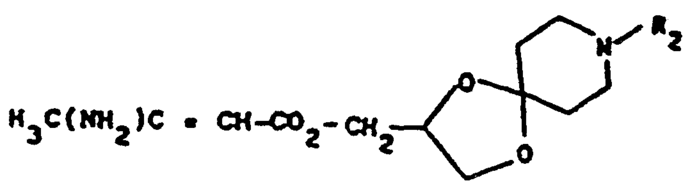

(III)

où $R_2$ est défini comme indiqué dans la revendication 1, et

b) les céto-esters de formule

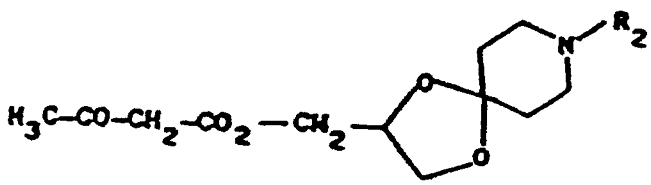

(VI)

où $R_2$ est défini comme indiqué dans la revendication 1.

Revendications pour les Etats contractants suivants : AT, ES, GR

1. Procédé de préparation d'un dérivé ester hétérocyclique dissymétrique de l'acide 1,4-dihydropyridine-3,5-dicarboxylique choisi parmi l'ensemble comprenant
(i) les esters répondant à la formule générale

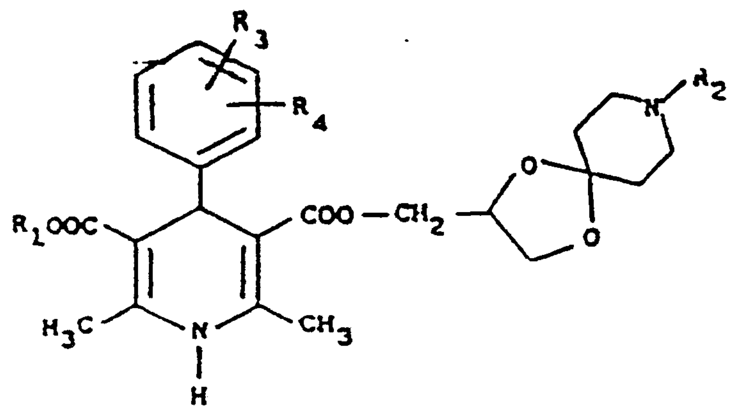

(I)

dans laquelle
$R_1$ représente un groupe alkyle en $C_1$-$C_4$;
$R_2$ représente un groupe alkyle en $C_1$-$C_4$, un groupe benzyle, un groupe benzoyle ou un groupe phényle éventuellement substitué par un ou plusieurs groupe alcoxy en $C_1$-$C_4$, alkyle en $C_1$-$C_4$, cyano, nitro, hydroxy, trifluorométhyle ou par un ou plusieurs atomes d'halogène ; $R_3$ et $R_4$, identiques ou différents, représentent chacun un atome d'hydrogène, un groupe nitro ou un atome de chlore;
(ii) leurs isomères optiques et diastéréoisomères; et,
(iii) les sels d'addition correspondants, ledit procédé étant caractérisé en ce que l'on fait réagir un benzylidène-acétoacétate de formule

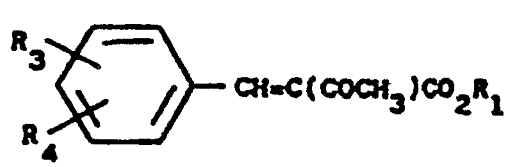

(II)

où $R_1$, $R_3$ et $R_4$ sont définis comme indiqué ci-dessus, avec un aminocrotonate de formule

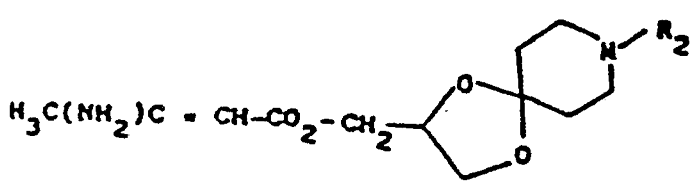

(III)

où $R_2$ est défini comme indiqué ci-dessus.

2. Procédé suivant la revendication 1, caractérisé en ce que l'on fait réagir environ 1 mole de benzylidèneacétoacétate de formule II avec environ 1 mole d'aminocrotonate de formule III dans un solvant organique polaire, notamment un alcanol en $C_1$-$C_4$, pendant 1 à 24 h, à une température comprise entre la température ambiante et la température de reflux du milieu réactionnel.

3. Procédé suivant la revendication 1, caractérisé en ce que le composé aminocrotonate de formule III est obtenu par

1°) réaction d'un alcool de formule

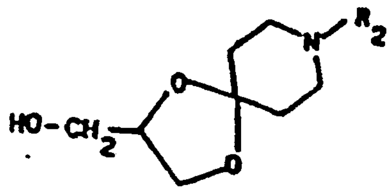

(IV)

où $R_2$ est défini comme indiqué dans la revendication 1, avec l'acide de Meldrum acétylé de formule

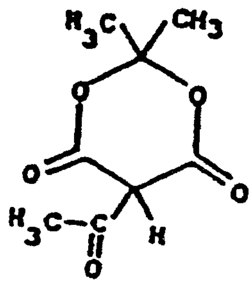

(V)

dans un solvant aromatique (notamment le benzène, le toluène, les xylènes) à une température comprise entre la température ambiante (15-20° C) et la température de reflux du milieu réactionnel, pendant 0,2 à 4 h, pour obtenir un cétoester nouveau de formule

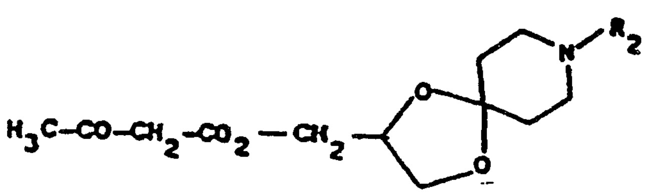

(VI)

où $R_2$ est défini comme indiqué dans la revendication 1, et

2°) traitement du dérivé de formule VI ainsi obtenu au moyen de $NH_3$ dans un solvant chloré (notamment $CH_2Cl_2$,$CHCl_3$) pendant 1 à 24 heures.

4. Procédé de préparation d'un dérivé ester dissymétrique de l'acide 1,4-dihydropyridine-3,5-dicarboxylique de formule I suivant la revendication 1, caractérisé en ce que l'on fait réagir un diol de formule

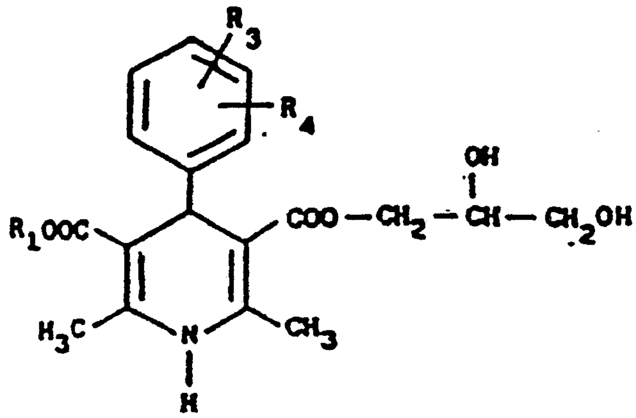

(VII)

où $R_1$, $R_3$ et $R_4$ sont définis comme indiqué dans la revendication 1, avec une pipéridinone de formule

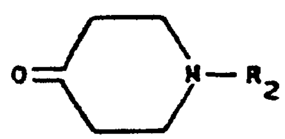

(VIII)

où $R_2$ est défini comme indiqué dans la revendication 1.

5. Procédé suivant la revendication 4, caractérisé en ce que les deux fonctions OH du diol de formule VII sont protégées.

6. Procédé suivant la revendication 4, caractérisé en ce que l'on fait réagir environ 1 mole de diol de formule VII avec environ 1 mole de pipéridinone de formule VIII, dans un solvant polaire en présence d'un acide minéral ou organique, à une température comprise entre la température ambiante et la température de reflux du milieu réactionnel, pendant 1 à 12 heures.

7. Procédé suivant la revendication 4, caractérisé en ce que ledit diol de formule VII est obtenu par ouverture du cycle dioxolannyle d'un composé de formule

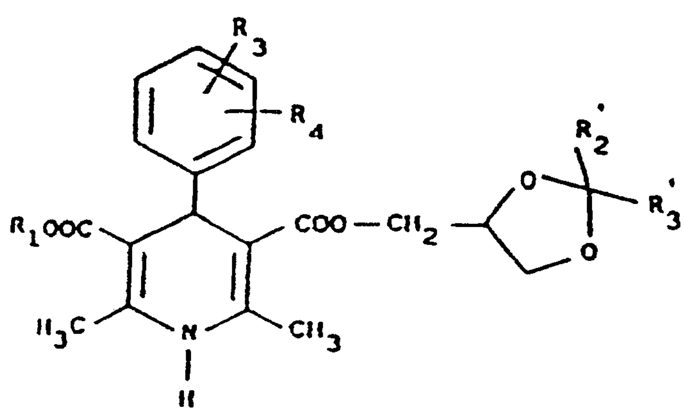

(IX)

où $R_1$, $R_3$ et $R_4$ sont définis comme indiqué dans la revendication 1, et $R'_2$ et $R'_3$ représentent chacun H ou un groupe alkyle en $C_1$-$C_4$, en milieu acide.

8. Procédé de préparation d'un énantiomère de formule (1) suivant la revendication 1, caractérisé en ce que
1°) on fait réagir une dihydropyridine de formule

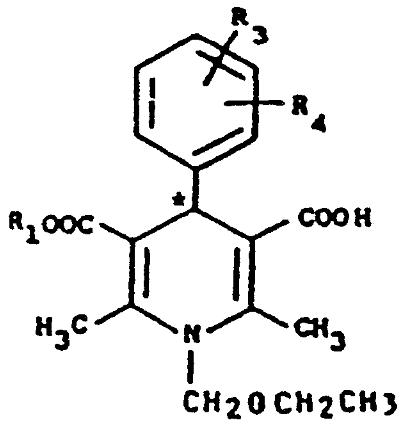

(X)

où $R_1$, $R_3$ et $R_4$ sont définis comme indiqué dans la revendication 1, et le carbone noté * à la configuration R ou la configuration S, avec le carbonyldiimidazole;

2°) sans isoler le dérivé intermédiaire formé, on soumet le milieu réactionnel ainsi obtenu à l'action d'un alcool de formule

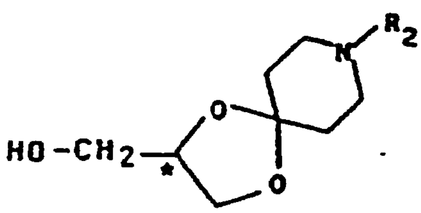

(IV')

où $R_2$ est défini comme dans la revendication 1 et le carbone noté * à la configuration R ou la configuration S, en présence d'une base organique forte, pour obtenir un composé de formule

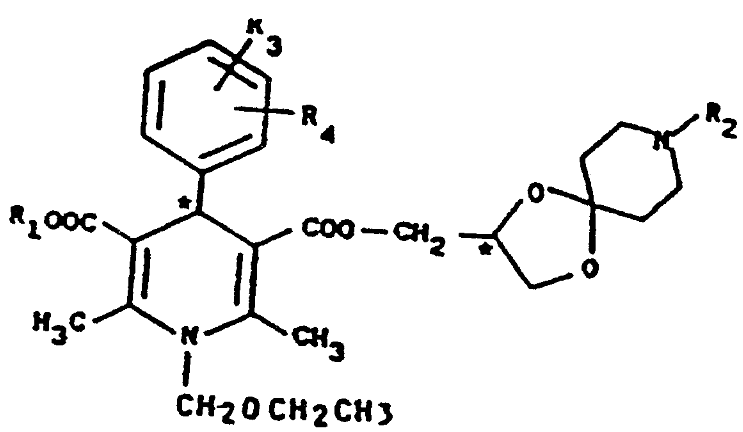

(XI)

où $R_1$, $R_2$, $R_3$ et $R_4$ sont définis comme indiqué dans la revendication 1, et les carbones notés * ont une configuration R ou une configuration S, et,

3°) on traite le composé de formule XI ainsi obtenu, en milieu acide fort dans un solvant organique, pour obtenir un énantiomère de formule

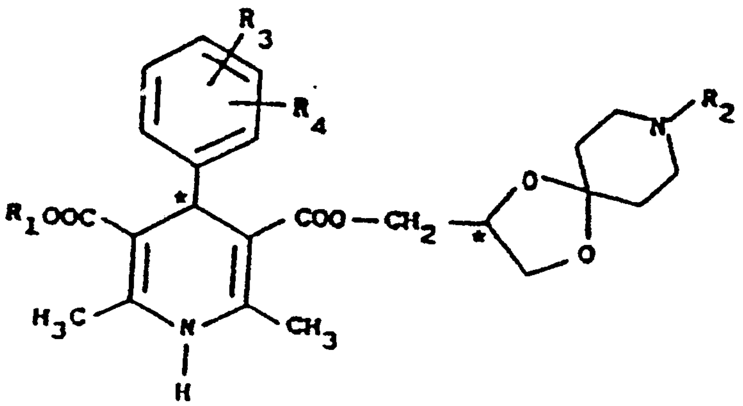

(I')

où $R_1$, $R_2$, $R_3$ et $R_4$ sont définis comme indiqué dans la revendication 1, et les carbones notés * ont une configuration R ou une configuration S.

9. Procédé suivant la revendication 8, caractérisé en ce que
   - au stade 1°, on fait réagir environ 1 mole de dihydropyridine de formule X avec environ 1 mole de carbonyldiimidazole, dans un solvant organique inerte, pendant 0,5 à 4 heures, à une température comprise entre la température ambiante et environ 80° C, et
   - au stade 2°, on fait réagir le milieu réactionnel résultant avec environ 1 mole d'un alcool de formule IV' dans une base organique forte, pendant 1 à 10 heures, à une température comprise entre environ 40° C et environ 150° C.

**Claims**

1. An asymmetrical heterocyclic ester derivative of 1,4-dihydropyridine-3,5-dicarboxylic acid, which is selected from the group comprising:
   (i) the esters corresponding to the general formula:

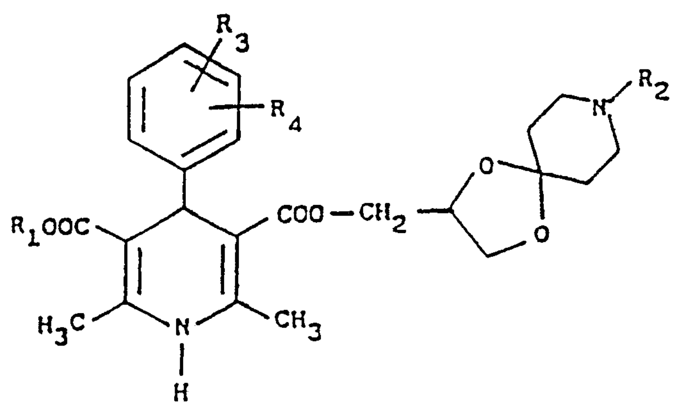

(I)

in which:
$R_1$ represents a $C_1$-$C_4$ alkyl group, $R_2$ represents a $C_1$-$C_4$ alkyl group, a benzyl group, a benzoyl group or a phenyl group optionally substituted by one or more $C_1$ - $C_4$ alkoxy, $C_1$-$C_4$ alkyl, cyano, nitro, hydroxyl or trifluoromethyl groups or by one or more halogen atoms, and $R_3$ and $R_4$, which are identical or different, each represent a hydrogen atom, a nitro group or a chlorine atom;
(ii) their optical isomers and diastereoisomers; and
(iii) the corresponding addition salts.

2. A derivative according to claim 1, wherein $R_2$ is $CH_3$, $CH_2C_6H_5$, $COC_6H_5$, $C_6H_5$, 4-methoxyphenyl, 3,4-dimethoxyphenyl, 4-fluorophenyl, 2-cyanophenyl, 4-cyanophenyl, 4-nitrophenyl, 3-trifluoromethylphenyl, 4-trifluoromethylphenyl, 4-chlorophenyl, 2,4-dichlorophenyl, 4-hydroxyphenyl or 4-methylphenyl.

3. A derivative according to claim 1, wherein $R_3$ and $R_4$ form the 3-nitrophenyl group or the 2,3-dichlorophenyl group with the phenyl nucleus to which they are bonded.

4. A compound according to claim 1, which is methyl (8-aza-8-(4-chlorophenyl)-1,4-dioxaspiro[4,5]decan-2-yl) methyl 1,4-dihydro-2,6-dimethyl-4-(3-nitrophenyl)pyridine-3,5-dicarboxylate.

5. A compound according to claim 1, which is methyl (8-aza-8-(4-chlorophenyl)-1,4-dioxaspiro[4,5] decan-2-yl) methyl 1,4-dihydro-2,6-dimethyl-4-(3-nitrophenyl)pyridine-3,5-dicarboxylate (R-(R*,S*)).

6. A compound according to claim 1, which is methyl (8-aza-8-(4-chlorophenyl)-1,4-dioxaspiro[4,5] decan-2-yl) methyl 1,4-dihydro-2,6-dimethyl-4-(3-nitrophenyl)pyridine-3,5-dicarboxylate (S-(R*,R*)).

7. A compound according to claim 1, which is methyl (8-aza-8-(4-chlorophenyl)-1,4-dioxaspiro[4,5] decan-2-yl) methyl 1,4-dihydro-2,6-dimethyl-4-(2,3-dichlorophenyl) pyridine-3,5-dicarboxylate.

8. A compound according to claim 1, which is methyl (8-aza-8-(2,4-dichlorophenyl)-1,4-dioxaspiro[4,5] decan-2-yl) methyl 1,4-dihydro-2,6-dimethyl-4-(3-nitrophenyl)pyridine-3,5-dicarboxylate.

9. A therapeutic composition which contains, in association with a physiologically acceptable excipient, at least one compound selected from the group comprising the derivatives of the formula I according to claim 1, their enantiomers and diastereoisomers and the corresponding addition salts.

10. Use of a substance selected from the group comprising (i) the compounds of the formula I according to claim 1, (ii) their enantiomers and diastereoisomers, and (iii) the corresponding non-toxic addition salts, in order to obtain an antihypertensive drug to be used in human therapy for the treatment of hypertension and cardiac insufficiencies.

11. A method for the preparation of a compound of the formula I according to claim 1, which comprises reacting a benzylideneacetoacetate of the formula:

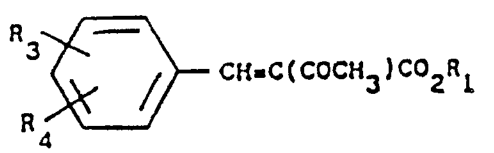

(II)

in which $R_1$, $R_3$ and $R_4$ are defined as indicated in claim 1, with an aminocrotonate of the formula :

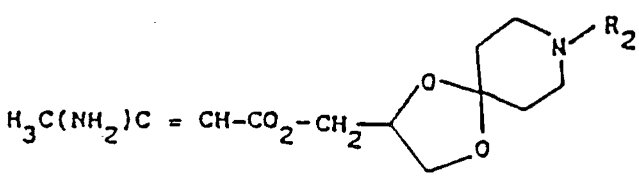

(III)

in wich $R_2$ is defined as indicated in claim 1, by bringing about 1 mol of II into contact with about 1 mol of III in a polar organic solvent, especially a $C_1$-$C_4$ alkanol, for 1 to 24 h, at a temperature between room temperature and the reflux temperature of the reaction medium, the compound III being obtained by the following method in particular:

1°) reaction of an alcohol of the formula:

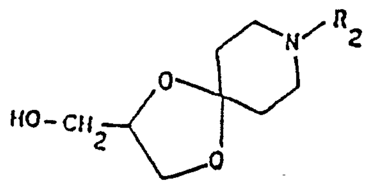

(IV)

in which $R_2$ is defined as indicated in claim 1, with acetylated Meldrum's acid of the formula:

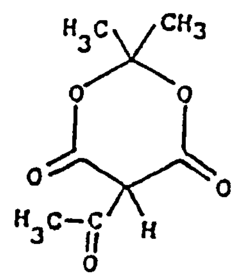

(V)

in an aromatic solvent (especially benzene, toluene or xylenes), at a temperature between room temperature (15-20 ° C) and the reflux temperature of the reaction medium, for 0.2 to 4 h, to give a novel ketoester of the formula:

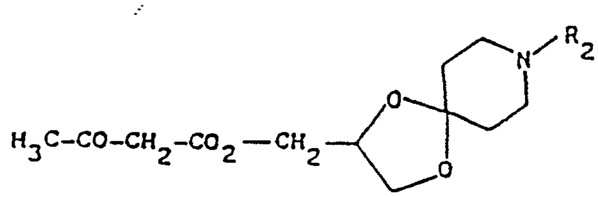

(VI)

in which $R_2$ is defined as indicated in claim 1, and
2° ) treatment of the resulting derivative of the formula VI with $NH_3$ in a chlorinated solvent (especially $CH_2Cl_2$ or $CHCl_3$) for 1 to 24 h.

**12.** A method for the preparation of a compound of the formula I according to claim 1, which comprises reacting a diol of the formula:

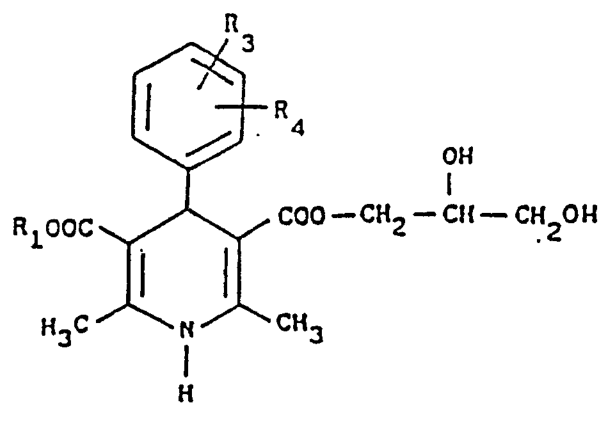

(VII)

in which $R_1$, $R_3$ and $R_4$ are defined as indicated in claim 1, with a piperidinone of the formula:

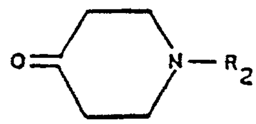

(VIII)

in which $R_2$ is defined as indicated in claim 1, it being possible for the OH functional groups or the said diol VII to be protected, if appropriate, during the ketalization in which about 1 mol of VII is reacted with about 1 mol of VIII in a polar solvent, in the presence of a mineral or organic acid, at a temperature between room temperature and the reflux temperature of the reaction medium, for 1 to 12 h, the said diol VII being obtained in particular by opening the dioxolanyl ring of a compound of the formula:

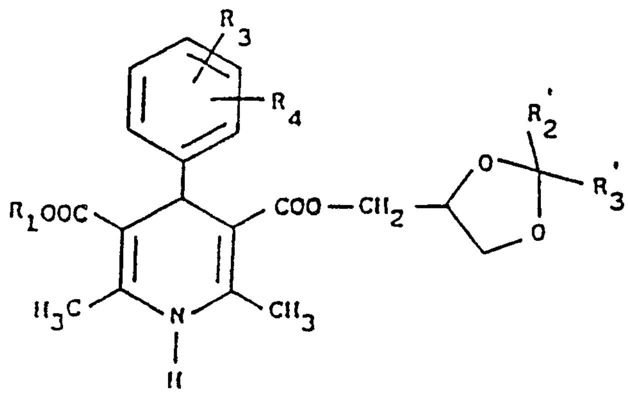

(IX)

in which $R_1$,$R_3$ and $R_4$ are defined as indicated in claim 1 and $R'_2$ and $R'_3$ each represent H or a $C_1$ - $C_4$ alkyl group, in an acid medium.

13. A method for the preparation of a compound of the formula I according to claim 1, wherein:
1°) about one mol of a dihydropyridine of the formula:

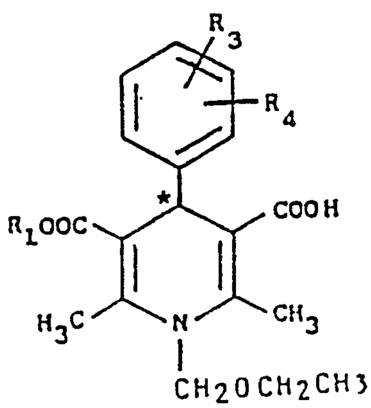

(X)

in which $R_1$, $R_3$ and $R_4$ are defined as indicated in claim 1 and the carbon marked * has the R configuration or the S configuration, is reacted with about one mol of carbonyldiimidazole, in an inert organic solvent, for 0.5 to 4 h, at a temperature between room temperature and about 80° C;
2°) without isolation of the intermediate derivative formed, the reaction medium is reacted with about one mol of an alcohol of the formula:

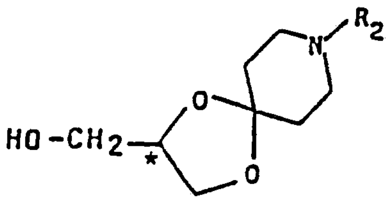

(IV')

in which $R_2$ is defined as indicated in claim 1 and the carbon marked * has the R configuration or the S configuration, in the presence of about one mol of a strong organic base, for 1 to 10 h, at a temperature of between about 40° C and about 150° C, to give a compound of the formula:

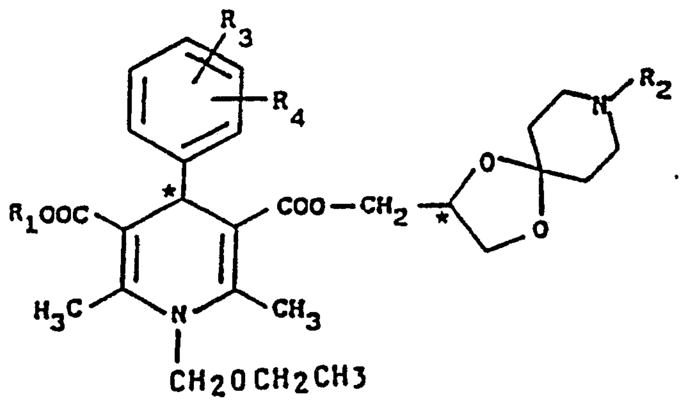

(XI)

in which $R_1$, $R_2$, $R_3$ and $R_4$ are defined as indicated in claim 1 and the carbons marked * have the R configuration or the S configuration; and

3°) the compound of the formula XI is treated in a strong acid medium, in an organic solvent, to give a compound of the formula:

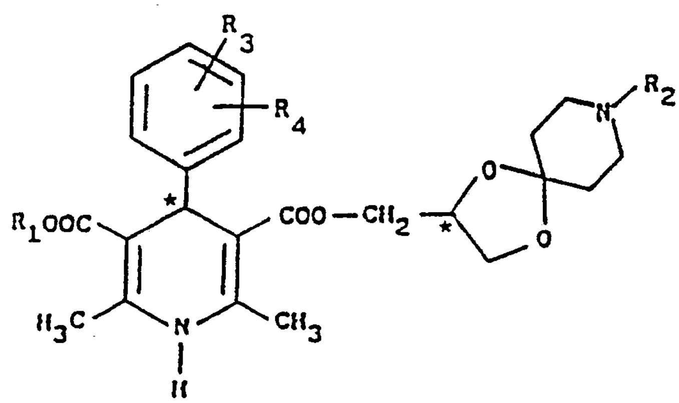

(I')

in which $R_1$, $R_2$, $R_3$ and $R_4$ are defined as indicated in claim 1 and the carbons marked * have the R configuration or the S configuration.

**14.** An intermediate involved in the synthesis of the compounds of the formula I according to claim 1, which is selected from the group comprising:

a) the aminocrotonates of the formula:

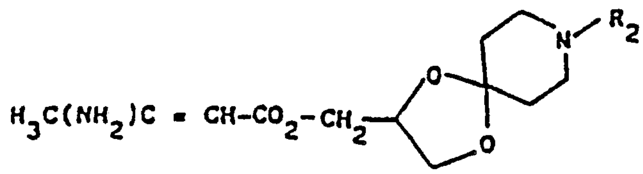

(III)

in which $R_2$ is defined as indicated in claim 1,

b) the ketoesters of the formula:

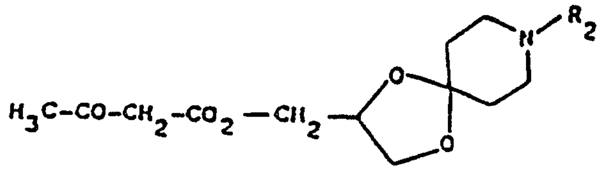

(VI)

in which $R_2$ is defined as indicated in claim 1.

Claims for the following Contracting States : AT, ES, GR

**1.** A method for the preparation of an asymmetrical heterocyclic ester derivative of 1,4-dihydropyridine-3,5-dicarboxylic acid, which is selected from the group comprising :

(i) the esters corresponding to the general formula:

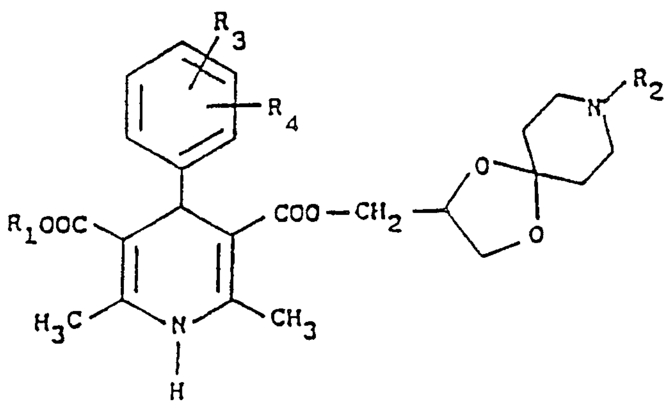

(I)

in which:

$R_1$ represents a $C_1$-$C_4$ alkyl group, $R_2$ represents a $C_1$-$C_4$ alkyl group, a benzyl group, a benzoyl group or a phenyl group optionally substituted by one or more $C_1$ - $C_4$ alkoxy, $C_1$-$C_4$ alkyl, cyano, nitro, hydroxyl or trifluoromethyl groups or by one or more halogen atoms, and $R_3$ and $R_4$, which are identical or different, each represent a hydrogen atom, a nitro group or a chlorine atom;

(ii) their optical isomers and diastereoisomers; and

(iii) the corresponding addition salts; said method comprising reacting a benzylideneacetoacetate of the formula:

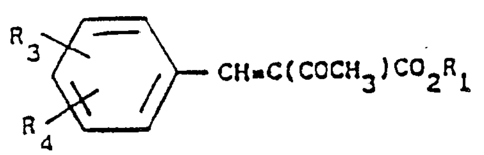

(II)

in which $R_1$, $R_3$ and $R_4$ are defined as indicated above with an aminocrotonate of the formula:

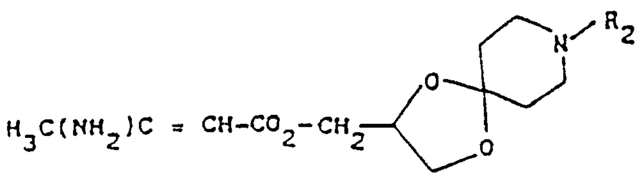

(III)

in which $R_2$ is defined as indicated above.

2. A method according to claim 1, which comprises bringing about 1 mol of II into contact with about 1 mol of III in a polar organic solvent, especially a $C_1$-$C_4$ alkanol, for 1 to 24 h, at a temperature between room temperature and the reflux temperature of the reaction medium.

3. A method according to claim 1, in which the aminocrotonate compound of the formula III is obtained by the following steps :
1°) reaction of an alcohol of the formula:

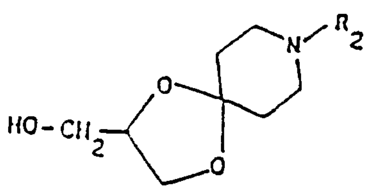

(IV)

in which $R_2$ is defined as indicated in claim 1, with acetylated Meldrum's acid of the formula:

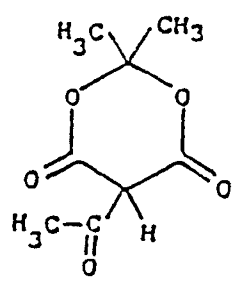

(V)

in an aromatic solvent (especially benzene, toluene or xylenes), at a temperature between room temperature (15-20° C) and the reflux temperature of the reaction medium, for 0.2 to 4 h, to give a novel ketoester of the formula:

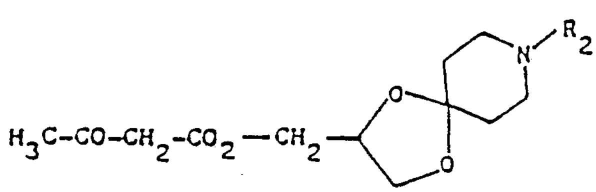

(VI)

in which $R_2$ is defined as indicated in claim 1, and
2°) treatment of the resulting derivative of the formula VI with $NH_3$ in a chlorinated solvent (especially $CH_2Cl_2$ or $CHCl_3$) for 1 to 24 h.

4. A method for the preparation of an asymmetrical heterocyclic ester derivative of 1,4-dihydropyridine-3,5-dicarboxylic acid of the formula I according to claim 1, which comprises reacting a diol of the formula:

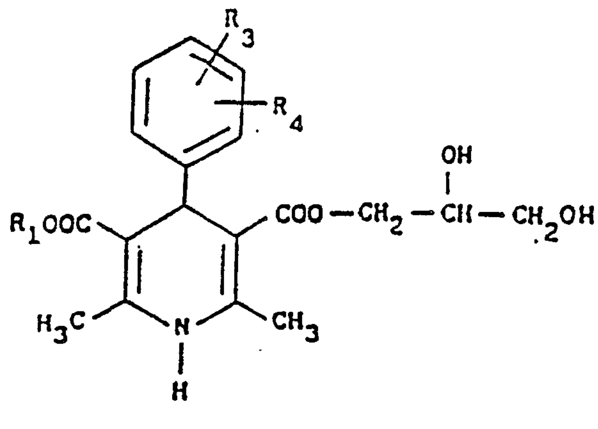

(VII)

in which $R_1$, $R_3$ and $R_4$ are defined as indicated in claim 1 with a piperidinone of the formula:

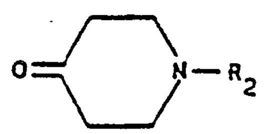

(VIII)

in which $R_2$ is defined as indicated in claim 1.

5. A method according to claim 4, in which both the OH functional groups of said diol compound of the formula VII are protected.

6. A method according to claim 4, in which about 1 mol of VII is reacted with about 1 mol of VIII in a polar solvent, in the presence of a mineral or organic acid, at a temperature between room temperature and the reflux temperature of the reaction medium, for 1 to 12 h.

7. A method according to claim 4, in which said diol compound of the formula VII is obtained in particular by opening the dioxolanyl ring of a compound of the formula:

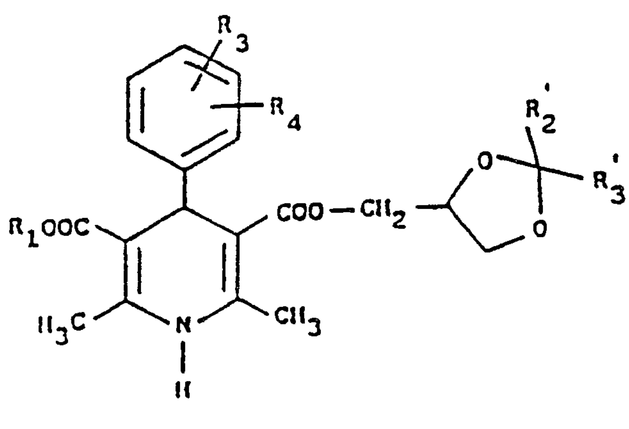

(IX)

in which $R_1$, $R_3$ and $R_4$ are defined as indicated in claim 1 and $R'_2$ and $R'_3$ each represent H or a $C_1$-$C_4$ alkyl group, in an acid medium.

8. A method for the preparation of an enantiomer compound of the formula I according to claim 1,

wherein:

1°) a dihydropyridine of the formula:

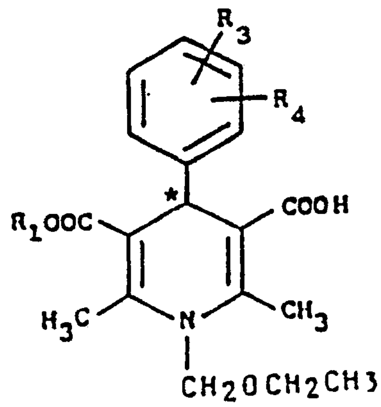

(X)

in which $R_1$, $R_3$ and $R_4$ are defined as indicated in claim 1 and the carbon marked * has the R configuration or the S configuration, is reacted with carbonyldiimidazole,

2°) without isolation of the intermediate derivative formed, the reaction medium is reacted with an alcohol of the formula:

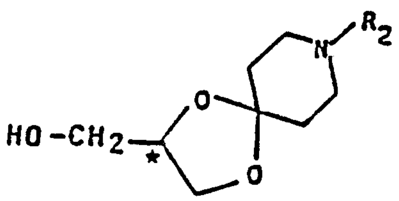

(IV')

in which $R_2$ is defined as indicated in claim 1 and the carbon marked * has the R configuration or the S configuration, in the presence of a strong organic base to give a compound of the formula:

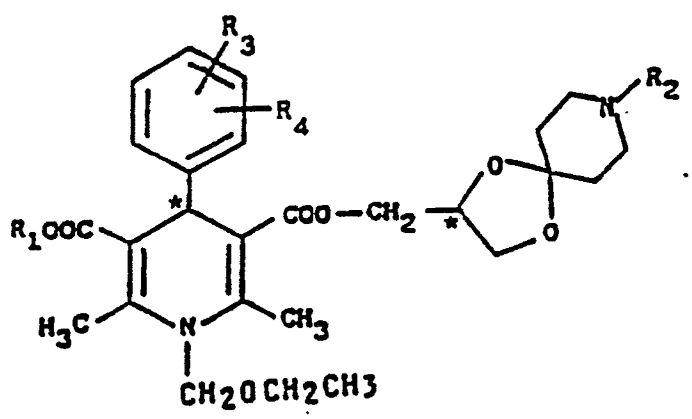

(XI)

in which $R_1$, $R_2$, $R_3$ and $R_4$ are defined as indicated in claim 1 and the carbons marked * have the R configuration or the S configuration; and

3°) The compound of the formula XI is treated in a strong acid medium, in an organic solvent, to give an enantiomer compound of the formula:

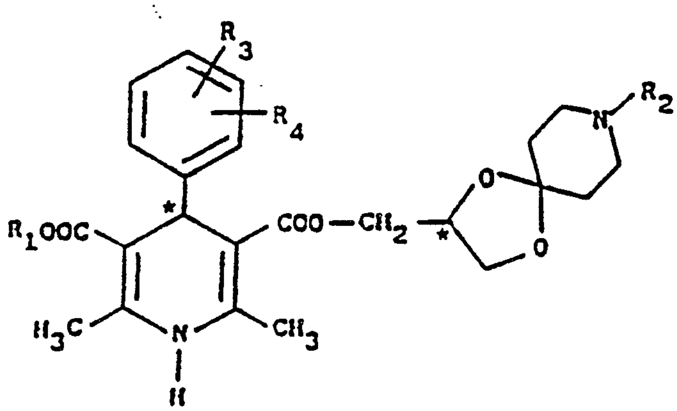

(I')

in which $R_1$, $R_2$, $R_3$ and $R_4$ are defined as indicated in claim 1, and the carbons marked * have the R configuration or the S-configuration.

9. A method according to claim 8, wherein :
   - in step 1°, about one mole of the dihydropyridine compound of the formula X is reacted with about one mole of carbonyldiimidazole, in an inert organic solvent, for 0.5 to 4 h, at a temperature between room temperature and about 80°C, and
   - in step 2°, the reaction medium thus obtained is reacted with about one mole of an alcohol of the formul IV', in a strong organic base, for 1 to 10 h, at a temperature of between about 40°C and about 150°C.

**Ansprüche**

1. Dissymetrische heterocyclische Esterderivate der 1,4-Dihydropyridin-3,5-dicarbonsäure, dadurch gekennzeichnet, daß sie ausgewählt sind aus der Gruppe, die umfaßt
   (i) die Ester gemäß der allgemeinen Formel

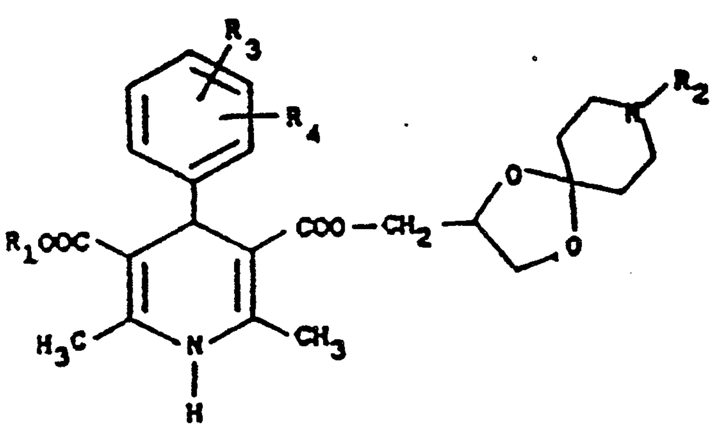

(I)

   in der $R_1$ eine Alkylgruppe mit 1 bis 4 Kohlenstoffatomen darstellt, $R_2$ eine Alkylgruppe mit 1 bis 4 Kohlenstoffatomen, eine Benzylgruppe, eine Benzoylgruppe oder eine Phenylgruppe ist, ggf. substituiert durch eine oder mehrere Alkoxygruppen mit 1 bis 4 Kohlenstoffatomen, Alkylgruppen mit 1 bis 4 Kohlenstoffatomen, Cyano-, Nitro-, Hydroxy-, Trifluormethyl-Gruppen oder durch ein oder mehrere Halogenatome, sowie $R_3$ und $R_4$, gleich oder verschieden, jeweils ein Wasserstoffatom, eine Nitrogruppe oder ein Chloratom bedeuten,
   (ii) deren optische Isomeren und Diastereoisomeren und
   (iii) korrespondierenden Additionssalze.

2. Derivate gemäß Anspruch 1,

dadurch gekennzeichnet,
daß $R_2$ bedeutet: $CH_3$, $CH_2C_6H_5$, $COC_6H_5$, $C_6H_5$, 4-Methoxyphenyl, 3,4-Dimethoxyphenyl, 4-Fluorphenyl, 2-Cyanophenyl, 4-Cyanophenyl, 4-Nitrophenyl, 3-Trifluormethylphenyl, 4-Trifluormethylphenyl, 4-Chlorphenyl, 2,4-Dichlorphenyl, 4-Hydroxyphenyl, 4-Methylphenyl.

**3.** Derivate gemäß Anspruch 1,
dadurch gekennzeichnet,
daß $R_3$ und $R_4$ mit dem Phenylrest, an den sie gebunden sind, die 3-Nitrophenyl- oder 2,3-Dichlorphenyl-Gruppe bilden.

**4.** Derivate gemäß Anspruch 1,
dadurch gekennzeichnet,
daß es sich handelt um den 1,4-Dihydro-2,6-dimethyl-4-(3-nitrophenyl)-pyridin-3,5-dicarbonsäure-methyl-und (8-aza-8-(4-chlorphenyl)-1,4-dioxa-spiro[4,5]-decan-2-yl)-methylester.

**5.** Derivate gemäß Anspruch 1,
dadurch gekennzeichnet,
daß es sich handelt um den 1,4-Dihydro-2,6-dimethyl-4-(3-nitrophenyl)-pyridin-3,5-dicarbonsäure-methyl-und (8-aza-8-(4-chlorphenyl)-1,4-dioxa-spiro[4,5]-decan-2-yl)-methylester(R-(R*,S*)).

**6.** Derivate gemäß Anspruch 1,
dadurch gekennzeichnet,
daß es sich handelt um den 1,4-Dihydro-2,6-dimethyl-4-(3-nitrophenyl)-pyridin-3,5-dicarbonsäure-methyl-und (8-aza-8-(4-chlorphenyl)-1,4-dioxa-spiro[4,5]-decan-2-yl)-methylester (S-(R*,R*)).

**7.** Derivate gemäß Anspruch 1,
dadurch gekennzeichnet,
daß es sich handelt um den 1,4-Dihydro-2,6-dimethyl-4-(2,3-dichlorphenyl)-pyridin-3,5-dicarbonsäure-methyl-und (8-aza-8-(4-chlorphenyl)-1,4-dioxa-spiro[4,5]-decan-2-yl)-methylester.

**8.** Derivate gemäß Anspruch 1,
dadurch gekennzeichnet,
daß es sich handelt um den 1,4-Dihydro-2,6-dimethyl-4-(3-nitrophenyl)-pyridin-3,5-dicarbonsäure-methyl-und (8-aza-8-(2,4-dichlorphenyl)-1,4-dioxa-spiro[4,5]-decan-2-yl)-methylester.

**9.** Therapeutische Zusammensetzung,
dadurch gekennzeichnet,
daß sie besteht aus einem Gemisch von einem physiologisch akzeptablen Träger mit wenigstens einer Verbindung ausgewählt aus der Gruppe der Derivate mit der Formel I gemäß Anspruch 1, deren enantiomeren Isomeren und deren Diastereoisomeren sowie den korrespondierenden Additionssalzen.

**10.** Verwendung einer Substanz ausgewählt aus
(i) den Verbindungen mit der Formel I gemäß Anspruch 1,
(ii) deren enantiomeren Isomeren und deren Diastereoisomeren und
(iii) den korrespondierenden nichttoxischen Additionssalzen zur Herstellung eines antihypertensiven Medikaments, bestimmt für die Humantherapie gegenüber Bluthochdruck und Herzinsuffizienzen.

**11.** Verfahren zur Herstellung einer Verbindung mit der Formel I gemäß Anspruch 1,
dadurch gekennzeichnet,
daß man ein Benzyliden-aceto-acetat der Formel

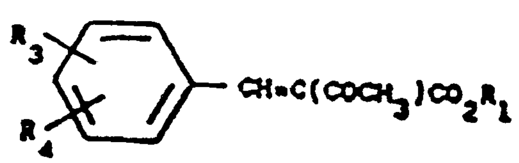

(II)

in der $R_1$, $R_3$ und $R_4$ der im Anspruch 1 angeführten Bedeutung entsprechen, mit einem Aminocrotonat der Formel

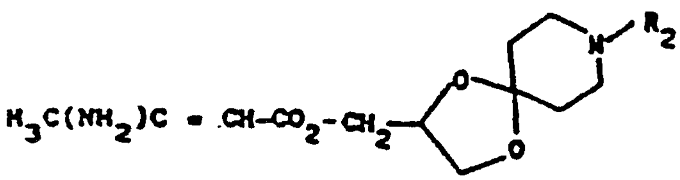

(III)

in der $R_2$ die im Anspruch 1 angeführte Bedeutung hat, zur Reaktion bringt in einem Verhältnis von etwa 1 Mol der Verbindung (II) mit etwa 1 Mol der Verbindung (III) in Gegenwart eines polaren organischen Lösungsmittels, insbesondere eines $C_1$-$C_4$-Alkanols, während 1 bis 24 Stunden bei einer Temperatur, die zwischen der Umgebungstemperatur und der Rückflußtemperatur der Reaktionsmischung liegt. wobei die Verbindung (III) vorzugsweise erhalten ist gemäß dem folgenden Reaktionsverlauf:

1. Umsetzung eines Alkohols mit der Formel

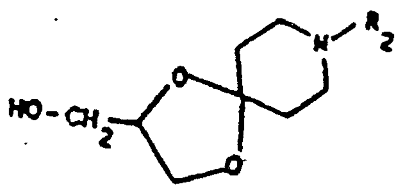

(IV)

in der $R_2$ die im Anspruch 1 angegebene Bedeutung hat, mit der acetylierten Meldrum-Säure mit der Formel

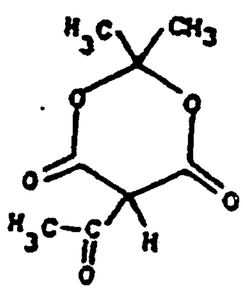

(V)

in einem aromatischen Lösungsmittel (besonders Benzol, Toluol, Xylol) bei einer Temperatur zwischen Umgebungstemperatur (15-20° C) und der Rückflußtemperatur der Reaktionsmischung während 0,2 bis 4 Stunden unter Gewinnung eines neuen Ketoesters mit der Formel

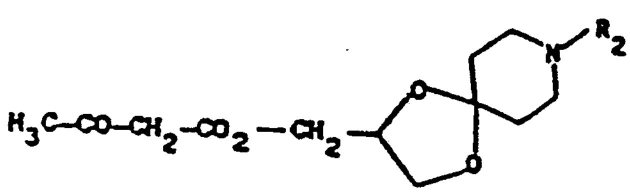

(VI)

in der $R_2$ der im Anspruch 1 angeführten Definition entspricht und

2. Behandlung des so erhaltenen Derivats mit der Formel VI mit $NH_3$ in einem chlorierten Lösungsmittel (besonders $CH_2Cl_2$, $CHCl_3$) während 1 bis 24 Stunden.

**12.** Verfahren zur Herstellung einer Verbindung mit der Formel I gemäß Anspruch 1,

dadurch gekennzeichnet,
daß man reagieren läßt ein Diol mit der Formel

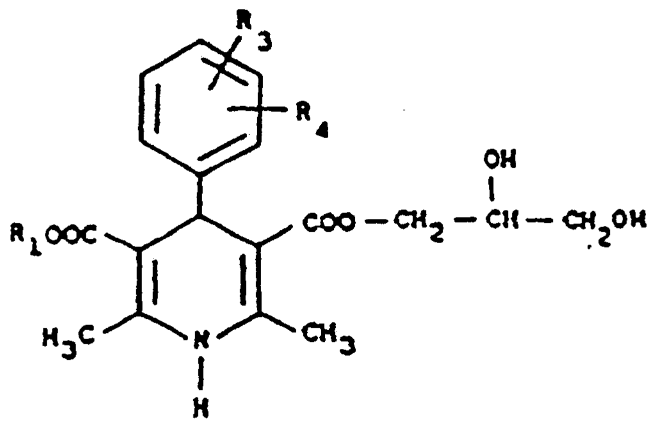

(VII)

in der $R_1$, $R_3$ und $R_4$ die im Anspruch 1 angeführte Bedeutung haben mit einem Piperidinon mit der Formel

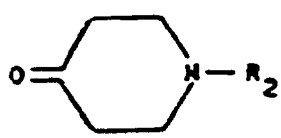

(VIII)

in der $R_2$ der im Anspruch 1 angeführten Definition entspricht und wobei die OH-Funktionen des Diols VII ggf. geschützt sind, während der Ketalisierung, die durchgeführt wird mit etwa 1 Mol von VII und etwa 1 Mol von VIII in einem polaren Lösungsmittel in Gegenwart einer Mineralsäure oder organischen Säure bei einer Temperatur zwischen der Umgebungstemperatur und der Rückflußtemperatur der Reaktionsmischung während 1 bis 12 Stunden, sowie das Diol VII insbesondere erhalten ist durch Öffnung des cyclischen Dioxolanringes einer Verbindung mit der Formel

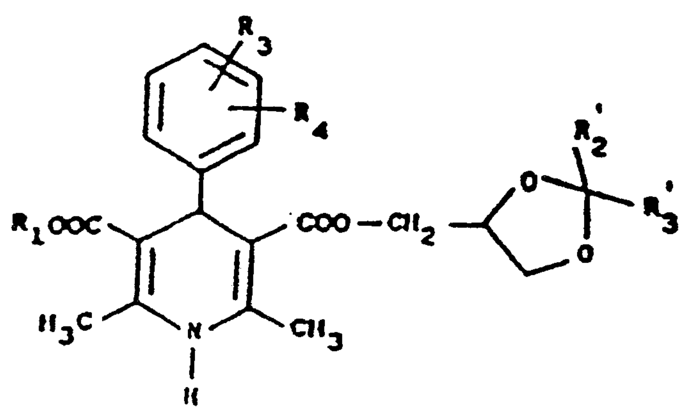

(IX)

in der $R_1$, $R_3$ und $R_4$ die Bedeutung gemäß Anspruch 1 aufweisen sowie $R'_2$ und $R'_3$ jeweils Wasserstoff oder eine Alkylgruppe mit 1 bis 4 Kohlenstoffatomen darstellt, in saurem Milieu.

**13.** Verfahren zur Herstellung einer Verbindung mit der Formel I gemäß Anspruch 1,
dadurch gekennzeichnet,
daß man
1. reagieren läßt etwa 1 Mol eines Dihydropyridins mit der Formel

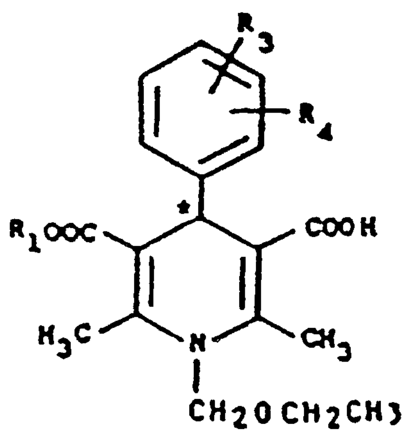

(X)

in der $R_1$, $R_3$ und $R_4$ die im Anspruch 1 angeführte Bedeutung haben und das durch * markierte Kohlenstoffatom die Konfiguration R oder die Konfiguration S hat, mit etwa 1 Mol Carbonyldiimidazol in einen inerten organischen Lösungsmittel während 0,5 bis 4 Stunden bei einer Temperatur zwischen Umgebungstemperatur und 80 °C`

2. ohne Isolierung des gebildeten Zwischenprodukts die Reaktionsmischung zur Umsetzung bringt mit etwa 1 Mol eines Alkohols mit der Formel

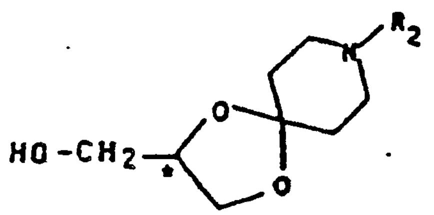

(IV')

in der $R_2$ der im Anspruch 1 angeführten Definition entspricht und das durch * markierte Kohlenstoffatom die Konfiguration R oder die Konfiguration S hat, in Gegenwart von etwa 1 Mol einer starken organischen Base während 1 bis 10 Stunden bei einer Temperatur zwischen etwa 40 °C und etwa 150 °C zur Gewinnung einer Verbindung mit der Formel

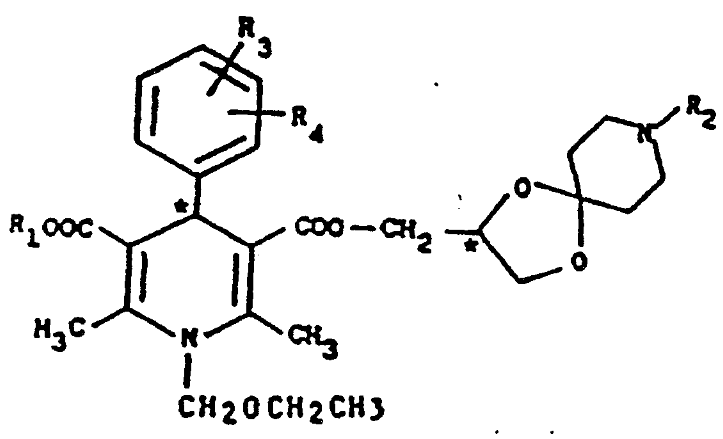

(XI)

in der $R_1$, $R_2$, $R_3$ und $R_4$ die im Anspruch 1 angeführte Bedeutung aufweisen und die mit * markierten Kohlenstoffatome eine Konfiguration R oder eine Konfiguration S haben, und

3. weiter behandelt die Verbindung mit der Formel XI in stark saurem Milieu in einem organischen Lösungsmittel zur Herstellung einer Verbindung mit der Formel

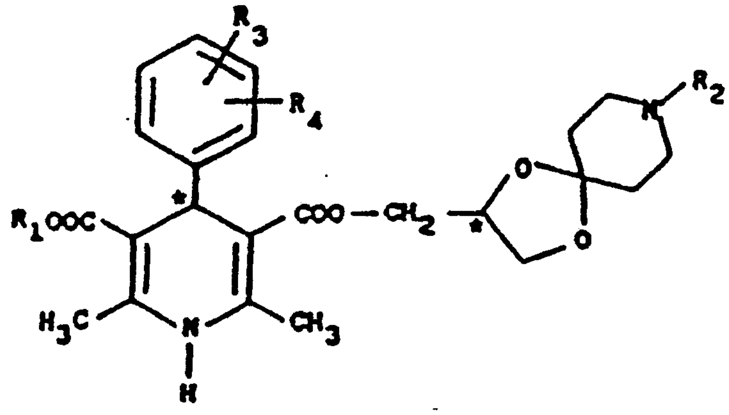

(I')

in der $R_1$, $R_2$, $R_3$ und $R_4$ der im Anspruch 1 angeführten Bedeutung entsprechen und die durch * markierten Kohlenstoffatome eine Konfiguration R oder eine Konfiguration S haben.

**14.** Zwischenprodukte, eingehend in die Synthese der Verbindungen mit der Formel I gemäß Anspruch 1, dadurch gekennzeichnet,
daß sie ausgewählt sind aus der Gruppe, die von
a) Aminocrotonaten mit der Formel

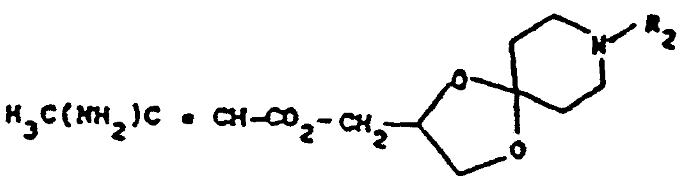

(III)

worin $R_2$ die im Anspruch 1 angeführte Bedeutung hat, und
b) Ketoestern mit der Formel

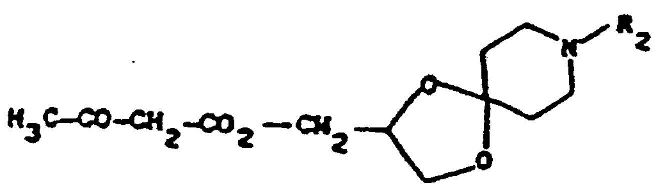

(VI)

in der $R_2$ der im Anspruch 1 angeführten Definition entspricht, gebildet wird.

Patentansprüche für folgende Vertragsstaaten : AT, ES, GR

**1.** Verfahren zur Herstellung eines dissymetrischen heterocyclischen Esterderivates der 1,4-Dihydropyridin-3,5-dircarbonsäure, ausgewählt aus der Gruppe, die umfaßt
(i) Ester entsprechend der allgemeinen Formel

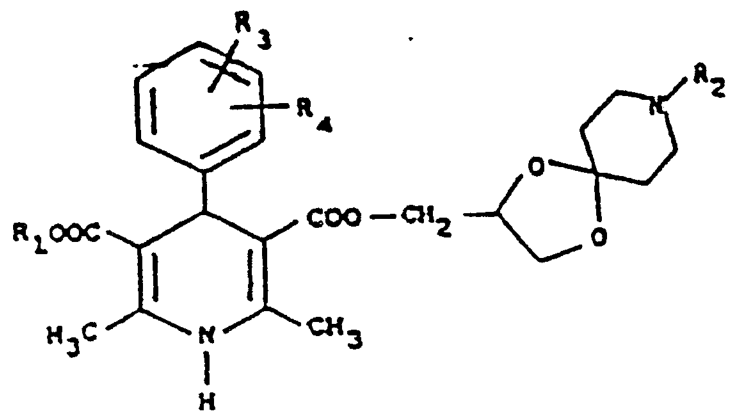

(1) .

in der $R_1$ eine Alkylgruppe mit 1 bis 4 Kohlenstoffatomen darstellt, $R_2$ eine Alkylgruppe mit 1 bis 4 Kohlenstoffatomen, eine Benzylgruppe, eine Benzoylgruppe oder eine Phenylgruppe ist, ggf. substituiert durch eine oder mehrere Alkoxygruppen mit 1 bis 4 Kohlenstoffatomen, Alkylgruppen mit 1 bis 4 Kohlenstoffatomen, Cyano-, Nitro-, Hydroxy-, Trifluormethyl-Gruppen oder durch ein oder mehrere Halogenatome sowie $R_3$ und $R_4$ , gleich oder verschieden, jeweils ein Wasserstoffatom, eine Nitrogruppe oder ein Chloratom bedeuten,
(ii) deren optische Isomeren und Diastereoisomeren und
(iii) korrespondierenden Additionssalze dadurch gekennzeichnet,
daß man miteinander zur Reaktion bringt ein Benzylidenacetoacetat mit der Formel

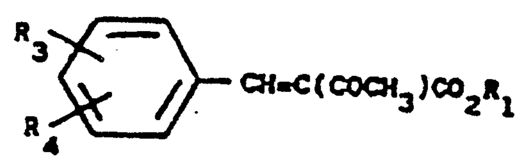

(II)

in der $R_1$, $R_3$ und $R_4$ der vorstehend angeführten Bedeutung entsprechen, mit einem Aminocrotonat der Formel

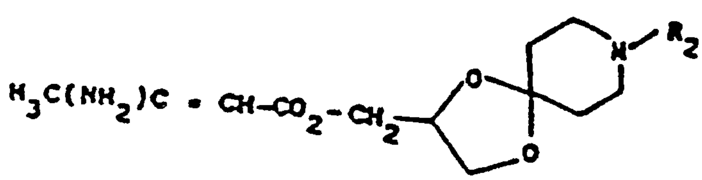

(III)

in der $R_2$ die weiter oben angeführte Bedeutung hat.

2. Verfahren gemäß Anspruch 1,
dadurch gekennzeichnet,
daß man miteinander reagieren läßt etwa 1 Mol des Benzylidenacetoacetats mit der Formel II mit etwa 1 Mol des Aminocrotonats mit der Formel III in einem polaren organischen Lösungsmittel, insbesondere einem Alkohol mit 1 bis 4 Kohlenstoffatomen, während 1 bis 24 Stunden bei einer Temperatur zwischen Umgebungstemperatur und der Rückflußtemperatur der Reaktionsmischung.

3. Verfahren gemäß Anspruch 1,
dadurch gekennzeichnet,
daß das Aminocrotonat mit der Formel III erhalten ist durch
1. Umsetzung eines Alkohols mit der Formel

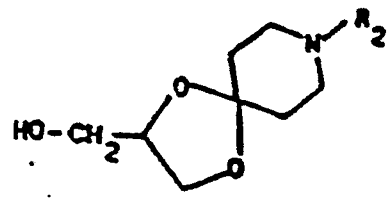

(IV)

in der $R_2$ die im Anspruch 1 angegebene Bedeutung hat, mit der acetylierten Meldrum-Säure mit der Formel

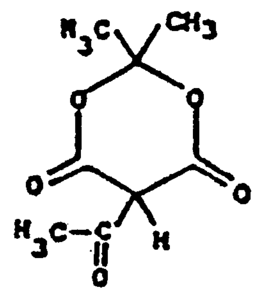

(V)

in einem aromatischen Lösungsmittel (besonders Benzol, Toluol, Xylol) bei einer Temperatur zwischen Umgebungstemperatur (15-20 °C) und der Rückflußtemperatur der Reaktionsmischung während 0,2 bis 4 Stunden unter Gewinnung eines neuen Ketoesters mit der Formel

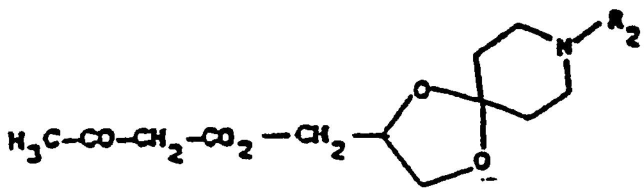

(VI)

in der $R_2$ der im Anspruch 1 angeführten Definition entspricht und
2. Behandlung des so erhaltenen Derivats mit der Formel VI mit $NH_3$ in einem chlorierten Lösungsmittel (besonders $CH_2Cl_2$, $CHCl_3$) während 1 bis 24 Stunden.

4. Verfahren zur Herstellung eines dissymetrischen Esterderivats mit der 1,4-Dihydropyridin-3,5-dicarbonsäure mit der Formel I gemäß Anspruch 1,
dadurch gekennzeichnet,
daß man miteinander zur Reaktion bringt 1 Diol mit der Formel

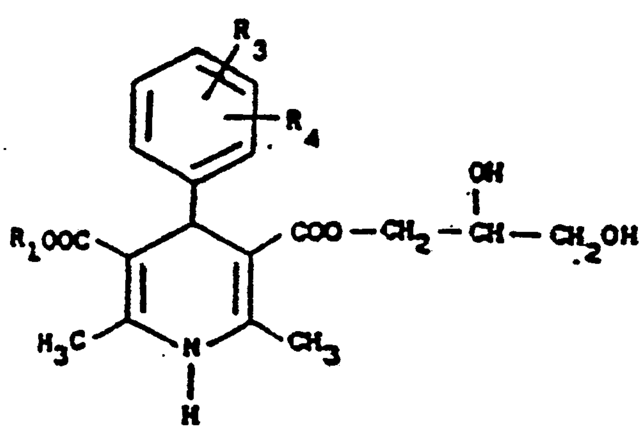

(VII)

in der $R_1$, $R_3$ und $R_4$ die im Anspruch 1 angeführte Bedeutung haben, mit einem Piperidinon mit der

Formel

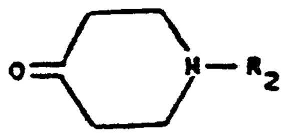

(VIII)

in der $R_2$ der im Anspruch 1 angeführten Definition entspricht.

5. Verfahren gemäß Anspruch 1,
dadurch gekennzeichnet,
daß die beiden OH-Funktionen des Diols der Formel VII geschützt sind.

6. Verfahren gemäß Anspruch 4,
dadurch gekennzeichnet,
daß man miteinander reagieren läßt etwa 1 Mol des Diols mit der Formel VII mit etwa 1 Mol des Piperidinons mit der Formel VIII in einem polaren Lösungsmittel in Gegenwart einer Mineralsäure oder einer organischen Säure bei einer Temperatur zwischen der Umgebungstemperatur und der Rückflußtemperatur der Reaktionsmischung während 1 bis 12 Stunden.

7. Verfahren gemäß Anspruch 4,
dadurch gekennzeichnet,
daß das Diol mit der Formel VII erhalten ist durch Öffnung des cyclischen Dioxolanringes einer Verbindung mit der Formel

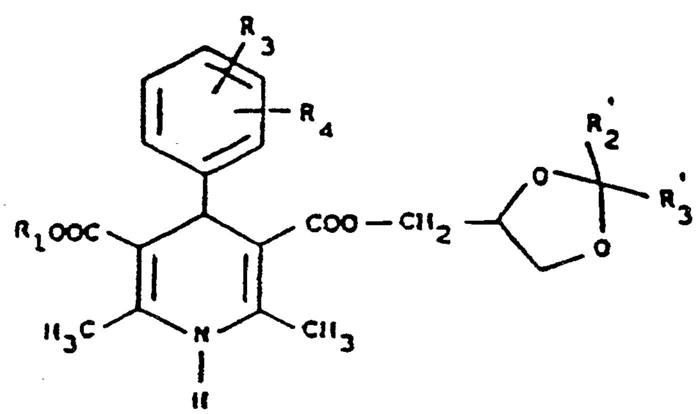

(IX)

in der $R_1$, $R_3$ und $R_4$ die Bedeutung gemäß Anspruch 1 aufweisen sowie $R'_2$ und $R'_3$ jeweils Wasserstoff oder eine Alkylgruppe mit 1 bis 4 Kohlenstoffatomen darstellt, in saurem Milieu.

8. Verfahren zur Herstellung eines Enantiomeren der Verbindung mit der Formel gemäß Anspruch 1,
dadurch gekennzeichnet, daß man
1. reagieren läßt ein Dihydropyridin mit der Formel

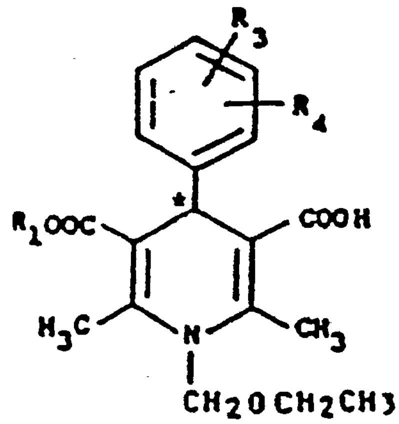

(X)

in der $R_1$, $R_3$ und $R_4$ die im Anspruch 1 angeführte Bedeutung haben und das durch * markierte Kohlenstoffatom die Konfiguration R oder die Konfiguration S hat mit Carbonyldiimidazol,
2. ohne Isolierung des gebildeten Zwischenprodukts die Reaktionsmischung zur Umsetzung bringt mit einem Alkohol mit der Formel

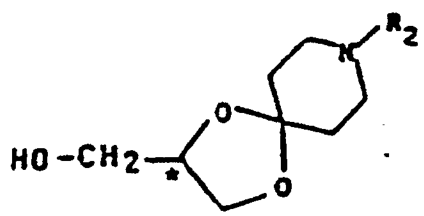

(IV')

in der $R_2$ der im Anspruch 1 angeführten Definition entspricht und das durch * markierte Kohlenstoffatom die Konfiguration R oder die Konfiguration S hat, in Gegenwart einer starken organischen Base zur Gewinnung einer Verbindung mit der Formel

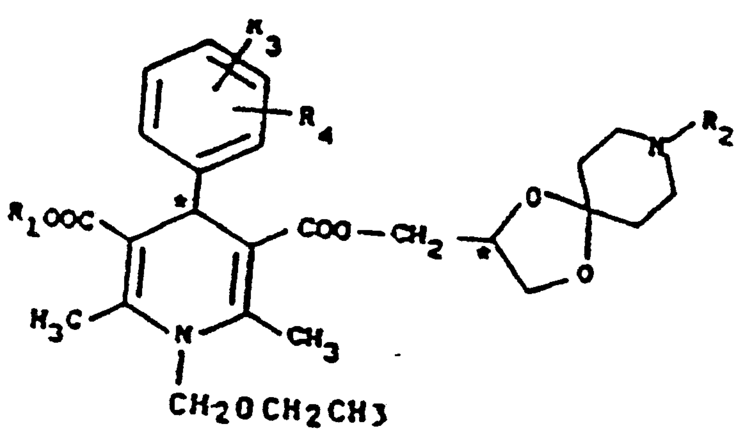

(XI)

in der $R_1$, $R_2$, $R_3$ und $R_4$ die im Anspruch 1 angeführte Bedeutung aufweisen und die mit * markierten Kohlenstoffatome eine Konfiguration R oder eine Konfiguration S haben, und
3. weiter behandelt die so erhaltene Verbindung mit der Formel XI in stark saurem Milieu in einem organischen Lösungsmittel zur Herstellung eines Enantiomeren mit der Formel

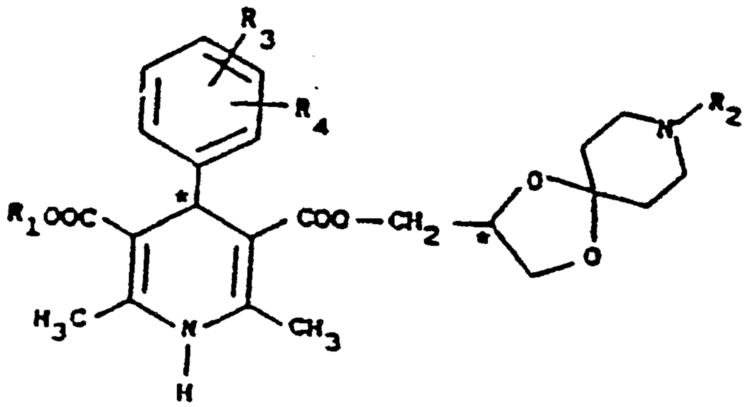

(I')

in der $R_1$, $R_2$, $R_3$ und $R_4$ der im Anspruch 1 angeführten Bedeutung entsprechen und die durch * markierten Kohlenstoffatome eine Konfiguration R oder eine Konfiguration S haben.

9. Verfahren gemäß Anspruch 8,
dadurch gekennzeichnet, daß man
- in der Stufe 1 etwa 1 Mol des Dihydropyridins mit der Formel X mit etwa 1 Mol Carbonyldiimidazol in einem inerten organischen Lösungsmittel während 0,5 bis 4 Stunden bei einer Temperatur zwischen Umgebungstemperatur und etwa 80° C zur Reaktion bringt und
- in der Stufe 2 die erhaltene Reaktionsmischung mit etwa 1 Mol eines Alkohols mit der Formel IV' in einer starken organischen Base während 1 bis 10 Stunden bei einer Temperatur zwischen etwa 40° C und etwa 150° C umsetzt.